# EUROPEAN PATENT SPECIFICATION

(11) **EP 3 004 062 B1**
(45) Date of publication and mention of the grant of the patent: **19.07.2017**
(21) Application number: 14729457.3
(22) Date of filing: 21.05.2014
(51) Int. Cl.: C07D 225/08

(54) **SUBSTITUTED AZADIBENZOCYCLOOCTYNE COMPOUNDS AND THEIR USE IN METAL-FREE CLICK REACTIONS**
SUBSTITUIERTE AZADIBENZOCYCLOOCTYNVERBINDUNGEN UND DEREN VERWENDUNG IN METALLFREIEN KLICKREAKTIONEN
COMPOSÉS AZADIBENZOCYCLOOCTYNE SUBSTITUÉS ET LEUR UTILISATION DANS DES RÉACTIONS « CLICK » SANS MÉTAL

(30) Priority: 24.05.2013 EP 13169208
(43) Date of publication of application: 13.04.2016
(73) Proprietor: SynAffix B.V., 5342 CC Oss (NL)
(72) Inventor: DEBETS, Marjoke Froukje, San Jose, CA 95134 (US); RUTJES, Floris Petrus Johannes Theodorus, NL-6605 DD Wijchen (NL); VAN HEST, Jan Cornelis Maria, NL-6533 AC Nijmegen (NL); VAN DELFT, Floris Louis, NL-6524 KZ Nijmegen (NL); VAN BERKEL, Sander Sebastiaan, 6663 HR Lent (NL)
(74) Representative: Nederlandsch Octrooibureau
(86) International application number: PCT/NL2014/050319
(87) International publication number: WO 2014/189370

(56) References cited:
- US-A1- 2012 029 186
- DEBETS M F ET AL: "Aza-dibenzocyclooctynes for fast and efficient enzyme PEGylation via copper-free (3+2) cycloaddition", CHEMICAL COMMUNICATIONS; [6015D], ROYAL SOCIETY OF CHEMISTRY, GB, vol. 46, no. 1, 1 January 2010 (2010-01-01), pages 97-99, XP002599643, ISSN: 1359-7345, DOI: 10.1039/B917797C [retrieved on 2009-11-06] cited in the application
- RYAN C. CHADWICK ET AL: "Synthesis of Conjugated Polymers Containing DIBAC-Derived Triazole Monomers", MACROMOLECULES, vol. 46, no. 24, 23 December 2013 (2013-12-23), pages 9593-9598, XP055128336, ISSN: 0024-9297, DOI: 10.1021/ma4021467

## Description

### Field of the invention

The present invention relates to substituted azadibenzocyclooctyne compounds and to a method for their preparation. The substituted azadibenzocyclooctyne compounds according to the invention may be used in metal-free click reactions. The invention therefore also relates to a method for the modification of a target molecule by reaction of an azadibenzocyclooctyne conjugate with a target molecule comprising a 1,3-dipole or a 1,3-(hetero)diene.

### Background of the invention

A revolutionary development in the rapidly expanding field of "chemical biology" is related to chemistry in living systems. Chemistry in living systems concerns chemical reactions that are mild in nature, yet so rapid and high-yielding that they work at about physiological pH, in water, and in the vicinity of biomolecular functionalities. Such reactions may be grouped under the term "bioorthogonal chemistry". In the field of bioorthogonal chemistry there are two main challenges: first, the development of suitable chemistry, and second, the application thereof in living organisms *(in vivo).*

In the field of chemistry, an enormous toolbox of chemical reactions is available that may be applied to the construction of complex organic molecules. However, the vast majority of such reactions can only be performed under strictly anhydrous conditions, in other words, in the complete absence of water. Although still a good minority of chemical reactions may be performed in, or in the presence of, water, most of these reactions can still only be applied *in vitro* because the interference of other compounds present in the living organism with the chemicals involved can not be excluded. At present, only a handful of chemical reactions is fully compatible with other functional groups present in the living organism.

An example of such a reaction is the cycloaddition of cyclic alkynes and azides, one of the reactions known as "click reactions". This reaction has become a versatile tool for bioorthogonal labeling and imaging of biomolecules (e.g. proteins, lipids, glycans and the like), proteomics and materials science. In essence, two separate molecular entities, one charged with an azide and one charged with a strained cycloalkyne, will spontaneously combine into a single molecule by a reaction called strain-promoted azide-alkyne cycloaddition (SPAAC). The power of SPAAC for bioorthogonal labeling lies in the fact that an isolated cyclic alkyne or azide is fully inert to biological functionalities, such as for example amines, thiols, acids or carbonyls, but in combination undergo rapid and irreversible cycloaddition leading to a stable triazole conjugate. For example, azido-modified proteins, obtained by expression in auxotrophic bacteria, genetic engineering or chemical conversion, can be cleanly labeled with biotin, fluorophores, PEG-chains or other functionalities upon simply stirring the azido-protein with a cyclooctyne conjugate. Moreover, the small size of azide has proven highly useful for application of SPAAC in the imaging of specific biomolecules by means of the chemical reporter strategy.

Apart from azides, cyclooctynes also show high reactivity with other dipoles, such as nitrones and nitrile oxides. For example, the strain-promoted alkyne-nitrone cycloaddition (SPANC) was applied for the *N*-terminal modification of proteins.

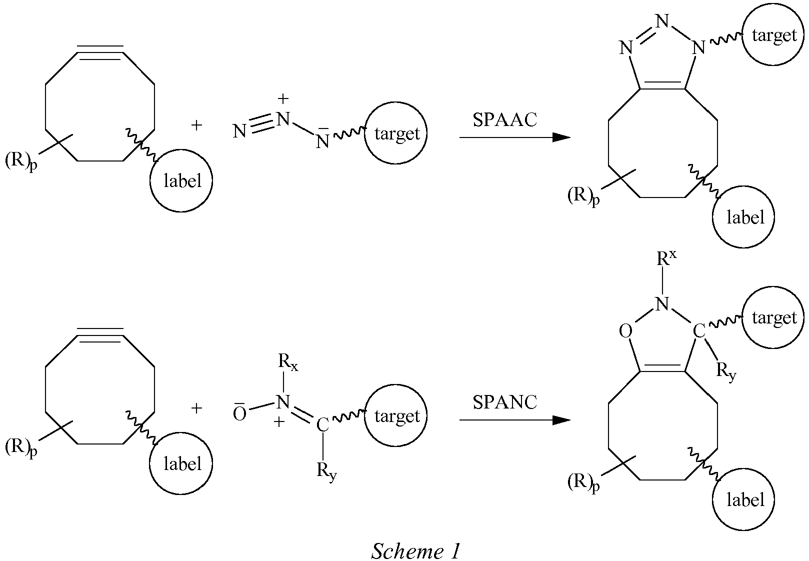

SPAAC and SPANC cycloaddition reactions (Scheme 1) proceed spontaneously, hence in the absence of a (metal) catalyst, and these and a select number of additional cycloadditions are also referred to as "metal-free click reactions".

Several cyclic alkynes and their application in bioorthogonal labeling are described in the prior art. US 2009/0068738 (Bertozzi et al.), incorporated by reference, relates to modified cycloalkyne compounds and their use in modifying biomolecules via a cycloaddition reaction that may be carried out under physiological conditions. The cycloaddition involves reacting a modified cycloalkyne, such as for example difluorinated cyclooctyne compounds DIFO, DIFO2 and DIFO3, with an azide moiety on a target biomolecule, generating a covalently modified biomolecule. It was observed that fluoride substitution has a strongly accelerating effect on the cycloaddition with azide. For example DIFO3 displays a significantly improved (30x faster) reaction rate constant of up to *k* = 0.076 M⁻¹ s⁻¹, versus a maximum of 0.0024 M⁻¹ s⁻¹ for non-fluorinated systems.

In WO 2011/136645 (van Delft and Rutjes et al.), incorporated by reference, a bicyclic compound wherein a cyclopropyl moiety is fused to a cyclooctyne moiety is disclosed. This fused cyclooctyne compound is used in metal-free click reactions. For example the cycloaddition with an organic azide in aqueous conditions proceeds with a rate constant in the range of *k* = 0.09 - 0.28 M⁻¹ s⁻¹ (depending on the solvent), whereas the rate constant for the cycloaddition with nitrones increases up to *k* = 1.25 M⁻¹ s⁻¹.

In a specific class of cyclic alkynes used in metal-free click reactions, the cyclooctyne moiety is fused to aryl groups (benzoannulated systems). An example of a benzoannulated system is disclosed in WO 2009/067663 (Boons et al.), incorporated by reference. The cycloaddition with azides of these dibenzocyclooctyne compounds DIBO (1) proceeds with a rate constant of *k =* 0.12 M⁻¹ s⁻¹.

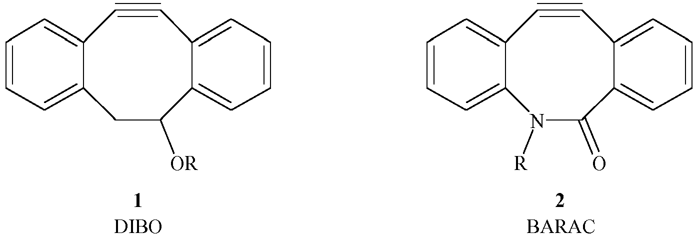

Another benzoannulated system, biarylazacyclooctynone BARAC (2), was reported by Bertozzi et al. (J. Am. Chem. Soc. 2010, 132, 3688 - 3690), incorporated by reference. By placing an amide functionality in the ring, the reaction kinetics of the cycloaddition of BARAC with azides is improved significantly (*k* = 0.96 M⁻¹ s⁻¹). A range of substituted derivatives of BARAC (Me, F, MeO) was also reported (J. Am. Chem. Soc. 2012, 134, 9199 - 9208) and the influence on reaction rate constant determined. However, only negligible difference in reactivity with azide was noticed (range for *k* = 0.9 - 1.2 M⁻¹ s⁻¹) by attachment of a single substituent on the aryl moiety, even for the strongly electron-withdrawing fluoride. An important disadvantage of BARAC and derivatives thereof is the relatively low stability, leading to short shelf-life (Org. Biomol. Chem., 2013, 11, 3436 - 3441). In addition, BARAC is known to be susceptible to Michael addition by thiols.

Azadibenzocyclooctyne DIBAC was developed earlier by Rutjes and van Delft et al. (Chem. Commun. 2010, 46, 97 - 99, incorporated by reference,) and shows somewhat lower reaction kinetics in the cycloaddition with azides (*k* = 0.31 M⁻¹ s⁻¹) with respect to BARAC, but in contrast DIBAC displays high stability and excellent shelf-life, and no Michael addition side-products. In addition, DIBAC can be readily synthesized in good yield by a variety of synthetic strategies. Based on this beneficial combination of properties, DIBAC (also often called DBCO or ADIBO) has become the standard cyclooctyne in research applications for copper-free click reactions with 1,3-dipoles.

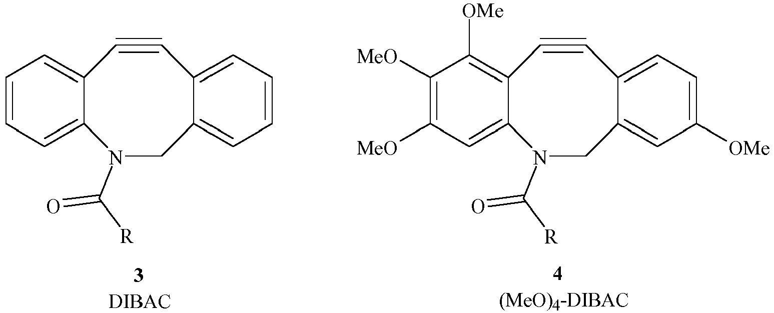

One paper by Starke et al. (Arkivoc 2010, 11, 350-359) describes the preparation and evaluation of the tetramethoxy-substituted DIBAC analogue (MeO)₄-DIBAC, but aryl substitution in this case led to significant decrease in reactivity (factor 40 with respect to plain DIBAC).

Another substituted DIBAC analogue is described in US 2012/0029186 (Popik et al.). In this analogue one or more C₁-C₁₂ organic groups, i.e. C₁ - C₁₂ hydrocarbon moieties, may be present on the aryl groups. However, no specific examples of these substituted DIBAC analogues are disclosed.

There exists a continuing need for novel, readily accessible and reactive bioorthogonal probes for use in metal-free click reactions, such as 1,3-dipolar cycloaddition with azides, nitrones and other 1,3-dipoles.

### Summary of the invention

The present invention relates to a compound of the Formula (**5**): 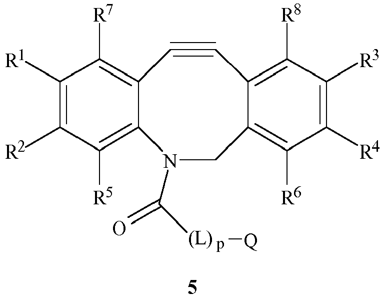 wherein:
R¹, R², R³, R⁴, R⁵, R⁶, R⁷ and R⁸ are independently selected from the group consisting of hydrogen, halogen, C₁ - C₁₂haloalkyl, -CN, -N₃, -NO₂, -NCX, -XCN, -N(R⁹)₂, -N⁺(R⁹)₃, -C(X)N(R⁹)₂, -C(X)R⁹, -C(X)XR⁹, -S(O)R⁹, -S(O)₂R⁹, -S(O)OR⁹, -S(O)₂OR⁹, -S(O)N(R⁹)₂, -S(O)₂N(R⁹)₂, -OS(O)R⁹, -OS(O)₂R⁹, -OS(O)OR⁹, -OS(O)₂OR⁹, -P(O)(R⁹)(OR⁹), -P(O)(OR⁹)₂, -OP(O)(OR⁹)₂, -XC(X)R⁹, -XC(X)XR⁹, -XC(X)N(R⁹)₂, -N(R⁹)C(X)R⁹, -N(R⁹)C(X)XR⁹ and -N(R⁹)C(X)N(R⁹)₂, wherein X is oxygen or sulfur and wherein R⁹ is independently selected from the group consisting of hydrogen, halogen, C₁ - C₂₄ alkyl groups, C₂ - C₂₄ (hetero)aryl groups, C₃ - C₂₄ alkyl(hetero)aryl groups and C₃ - C₂₄ (hetero)arylalkyl groups;
with the proviso that at least one of R¹, R², R³, R⁴, R⁵, R⁶, R⁷ and R⁸ is selected from the group consisting of halogen, C₁ - C₁₂ haloalkyl-CN, -N₃, -NO₂, -NCX, -XCN, -N(R⁹)₂, -N⁺(R⁹)₃, -C(X)N(R⁹)₂, -C(X)R⁹, -C(X)XR⁹, -S(O)R⁹, -S(O)₂R⁹, -S(O)OR⁹, -S(O)₂OR⁹, -S(O)N(R⁹)₂, -S(O)₂N(R⁹)₂, -OS(O)R⁹, -OS(O)₂R⁹, -OS(O)OR⁹, -OS(O)₂OR⁹, -P(O)(R⁹)(OR⁹), -P(O)(OR⁹)₂, -OP(O)(OR⁹)₂, -XC(X)R⁹, -XC(X)XR⁹, -XC(X)N(R⁹)₂, -N(R⁹)C(X)R⁹, -N(R⁹)C(X)XR⁹ and -N(R⁹)C(X)N(R⁹)₂, wherein X and R⁹ are as defined above;
p is 0 or 1;
L is a linking group selected from the group consisting of linear or branched C₁-C₂₀₀ alkylene groups, C₂-C₂₀₀ alkenylene groups, C₂-C₂₀₀ alkynylene groups, C₃-C₂₀₀ cycloalkylene groups, C₅-C₂₀₀ cycloalkenylene groups, C₈-C₂₀₀ cycloalkynylene groups, C₂-C₂₀₀ (hetero)arylene groups, C₃-C₂₀₀ alkyl(hetero)arylene groups, C₃-C₂₀₀ (hetero)arylalkylene groups, C₄-C₂₀₀ (hetero)arylalkenylene groups, C₅-C₂₀₀ (hetero)arylalkynylene groups, the alkylene groups, alkenylene groups, alkynylene groups, cycloalkylene groups, cycloalkenylene groups, cycloalkynylene groups, (hetero)arylene groups, alkyl(hetero)arylene groups, (hetero)arylalkylene groups, (hetero)arylalkenylene groups, (hetero)arylalkynylene groups optionally being substituted and/or optionally interrupted by one or more heteroatoms, preferably 1 to 100 heteroatoms, said heteroatoms preferably being selected from the group consisting of O, S, N and NR¹², wherein R¹² is independently selected from the group consisting of hydrogen, halogen, C₁ - C₂₄ alkyl groups, C₂ - C₂₄ (hetero)aryl groups, C₃ - C₂₄ alkyl(hetero)aryl groups and C₃ - C₂₄ (hetero)arylalkyl groups; and
Q is a functional group selected from the group consisting of hydrogen, halogen, R¹¹, -CH=C(R¹¹)₂, -C≡CR¹¹, -[C(R¹¹)₂C(R¹¹)₂O]_{q}-R¹¹ wherein q is in the range of 1 to 200, -CN, -N₃, -NCX, -XCN, -XR¹¹, -N(R¹¹)₂, -N⁺(R¹¹)₃, -C(X)N(R¹¹)₂, -C(R¹¹)₂XR¹¹, -C(X)R¹¹, -C(X)XR¹¹, -S(O)R¹¹, -S(O)₂R¹¹, -S(O)OR¹¹, -S(O)₂OR¹¹, -S(O)N(R¹¹)₂, -S(O)₂N(R¹¹)₂, -OS(O)R¹¹, -OS(O)₂R¹¹, -OS(O)OR¹¹, -OS(O)₂OR¹¹, -P(O)(R¹¹)(OR¹¹), -P(O)(OR¹¹)₂, -OP(O)(OR¹¹)₂, -Si(R¹¹)₃, -XC(X)R¹¹, -XC(X)XR¹¹, -XC(X)N(R¹¹)₂, -N(R¹¹)C(X)R¹¹, -N(R¹¹)C(X)XR¹¹ and -N(R¹¹)C(X)N(R¹¹)₂, wherein X is oxygen or sulfur and wherein R¹¹ is independently selected from the group consisting of hydrogen, halogen, C₁ - C₂₄ alkyl groups, C₂ - C₂₄ (hetero)aryl groups, C₃ - C₂₄ alkyl(hetero)aryl groups and C₃ - C₂₄ (hetero)arylalkyl groups.

The invention also relates to a conjugate wherein a compound according to Formula (5) is conjugated to a label via a functional group Q, and to the use of said conjugate for bioorthogonal labeling, imaging or modification of a target molecule. The invention further relates to a method for the modification of a target molecule, wherein a conjugate according to the invention is reacted with a compound comprising a 1,3-dipole or a 1,3-(hetero)diene.

### Description of the figures

Figure 1 shows the general synthetic route for the preparation of the azadibenzocyclooctyne compounds according to the invention.

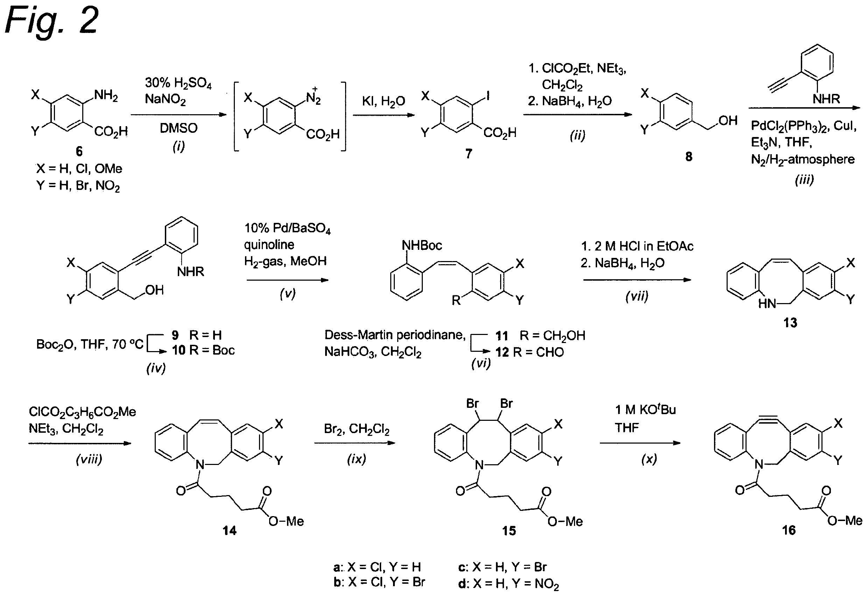

Figure 2 shows the synthetic route for the synthesis of several specific azadibenzocyclooctyne compounds **16a, 16b**, **16c** and **16d**.

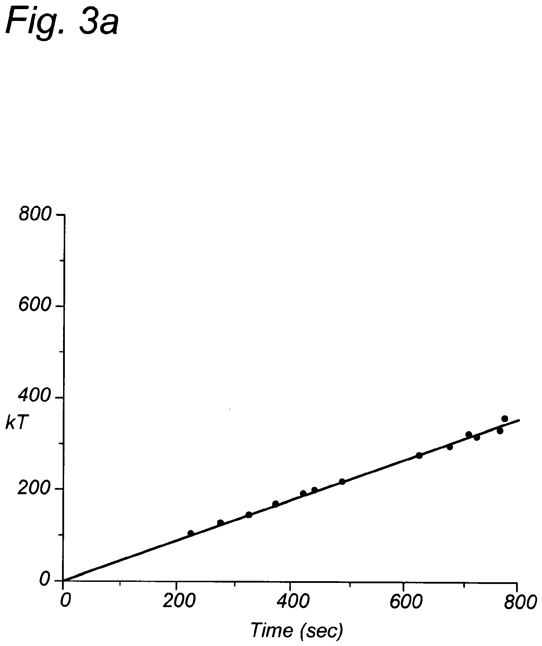

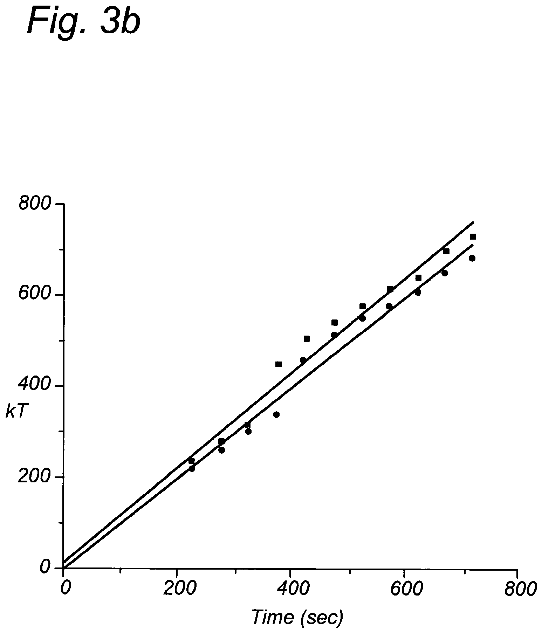

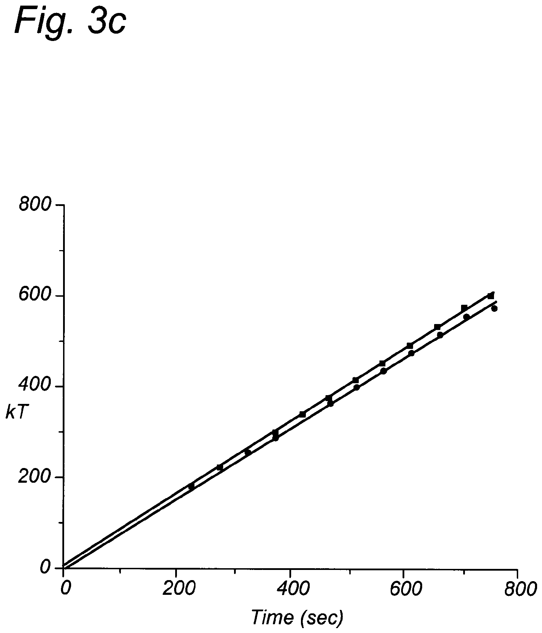

Figure 3 shows logarithmic plots of cycloaddition of benzyl azide with (a) DIBAC (**3**); (b) C1-DIBAC (**16a**) and (c) Br-DIBAC (**16c**).

### Detailed description of the invention

### Definitions

The verb "to comprise" as is used in this description and in the claims and its conjugations is used in its non-limiting sense to mean that items following the word are included, but items not specifically mentioned are not excluded.

In addition, reference to an element by the indefinite article "a" or "an" does not exclude the possibility that more than one of the element is present, unless the context clearly requires that there is one and only one of the elements. The indefinite article "a" or "an" thus usually means "at least one".

The compounds disclosed in this description and in the claims may be described as benzoannulated azacyclooctyne compounds, *i.e*. cyclooctyne compounds wherein two aromatic moieties are fused to the cyclooctyne moiety and the cyclooctyne contains a nitrogen.

The compounds disclosed in this description and in the claims may further exist as positional isomers. Unless stated otherwise, the description of any compound in the description and in the claims is meant to include positional isomers of DIBAC, with substituents on either or both of the two aromatic rings, as well as mixtures thereof.

An unsubstituted alkyl group has the general formula CₙH₂ₙ₊₁ and may be linear or branched. Unsubstituted alkyl groups may also contain a cyclic moiety, and thus have the concomitant general formula CₙH₂ₙ₋₁. Optionally, the alkyl groups are substituted by one or more substituents further specified in this document. Examples of suitable alkyl groups include methyl, ethyl, propyl, 2-propyl, t-butyl, 1-hexyl and 1-dodecyl.

A haloalkyl group has the general formula CₙY_{q}H_{(2n+1)-q}, wherein Y is selected from the group consisting of F, Cl, Br and I and wherein q = in the range of 1 - 25. A haloalkyl group may be linear or branched. Examples of suitable haloalkyl groups include trifluoromethyl (-CF₃), pentafluoroethyl (-CF₂CF₃), tribromomethyl (-CBr₃) and pentabromoethyl (-CF₂CF₃), dibromomethyl (-CHBr₂), dichloromomethyl (-CHCl₂), difluoromethyl (-CHF₂), bromomethyl (-CH₂Br), chloromomethyl (-CH₂Cl) and fluoromethyl (-CH₂F).

Unsubstituted alkenyl groups have the general formula CₙH₂ₙ₋₁, and may be linear or branched. Examples of suitable alkenyl groups include ethenyl, propenyl, isopropenyl, butenyl, pentenyl, decenyl, octadecenyl and eicosenyl. Unsubstituted alkenyl groups may also contain a cyclic moiety, and thus have the concomitant general formula CₙH₂ₙ₋₃. Optionally, the alkenyl groups may be substituted by one or more substituents further specified in this document.

Unsubstituted alkenes have the general formula CₙH₂ₙ whereas unsubstituted alkynes have the general formula CₙH₂ₙ₋₂. Optionally, the alkenes and alkynes may be substituted by one or more substituents further specified in this document.

Aryl groups comprise at least six carbon atoms (i.e. at least C₆) and may include monocyclic, bicyclic and polycyclic structures. Optionally, the aryl groups may be substituted by one or more substituents further specified in this document. Examples of aryl groups include groups such as for example phenyl, naphthyl and anthracyl.

Arylalkyl groups and alkylaryl groups comprise at least seven carbon atoms (i.e. at least C₇) and may include monocyclic and bicyclic structures. Optionally, the aryl groups may be substituted by one or more substituents further specified in this document. An arylalkyl group is for example benzyl and the like. An alkylaryl group is for example 4-t-butylphenyl and the like.

Heteroaryl groups comprise at least two carbon atoms (i.e. at least C₂) and one or more heteroatoms N, O, P or S. A heteroaryl group may have a monocyclic or a bicyclic structure. Optionally, the heteroaryl group may be substituted by one or more substituents further specified in this document. Examples of suitable heteroaryl groups include pyridinyl, quinolinyl, pyrimidinyl, pyrazinyl, pyrazolyl, imidazolyl, thiazolyl, pyrrolyl, furanyl, triazolyl, benzofuranyl, indolyl, purinyl, benzoxazolyl, thienyl, phospholyl and oxazolyl.

Heteroarylalkyl groups and alkylheteroaryl groups comprise at least three carbon atoms (i.e. at least C₃) and may include monocyclic and bicyclic structures. Optionally, the heteroaryl groups may be substituted by one or more substituents further specified in this document.

Where an aryl group is denoted as a (hetero)aryl group, the notation is meant to include an aryl group and a heteroaryl group. Similarly, an alkyl(hetero)aryl group is meant to include an alkylaryl group and a alkylheteroaryl group, and (hetero)arylalkyl is meant to include an arylalkyl group and a heteroarylalkyl group. A C₂ - C₂₄ (hetero)aryl group is thus to be interpreted as including a C₂ - C₂₄ heteroaryl group and a C₆ - C₂₄ aryl group. Similarly, a C₃ - C₂₄ alkyl(hetero)aryl group is meant to include a C₇ - C₂₄ alkylaryl group and a C₃ - C₂₄ alkylheteroaryl group, and a C₃ - C₂₄ (hetero)arylalkyl is meant to include a C₇ - C₂₄ arylalkyl group and a C₃ - C₂₄ heteroarylalkyl group.

Unless stated otherwise, alkyl groups, alkenyl groups, alkenes, alkynes, (hetero)aryl groups, (hetero)arylalkyl groups and alkyl(hetero)aryl groups may be substituted with one or more substituents selected from the group consisting of, C₂- C₁₂ alkenyl groups, C₂ - C₁₂ alkynyl groups, C₃ - C₁₂ cycloalkyl groups, C₅ - C₁₂ cycloalkenyl groups, C₈ - C₁₂ cycloalkynyl groups, C₁ - C₁₂ alkoxy groups, C₂ - C₁₂ alkenyloxy groups, C₂ - C₁₂ alkynyloxy groups, C₃ - C₁₂ cycloalkyloxy groups, halogens, amino groups, oxo and silyl groups, wherein the silyl groups can be represented by the formula (R¹⁰)₃Si-, wherein R¹⁰ is independently selected from the group consisting of C₁ - C₁₂ alkyl groups, C₂ - C₁₂ alkenyl groups, C₂ - C₁₂ alkynyl groups, C₃ - C₁₂ cycloalkyl groups, C₁ - C₁₂ alkoxy groups, C₂ - C₁₂ alkenyloxy groups, C₂ - C₁₂ alkynyloxy groups and C₃ - C₁₂ cycloalkyloxy groups, wherein the alkyl groups, alkenyl groups, alkynyl groups, cycloalkyl groups, alkoxy groups, alkenyloxy groups, alkynyloxy groups and cycloalkyloxy groups are optionally substituted, the alkyl groups, the alkoxy groups, the cycloalkyl groups and the cycloalkoxy groups being optionally interrupted by one of more hetero-atoms selected from the group consisting of O, N and S.

### Substituted azadibenzocyclooctyne compounds

In a first aspect, the present invention relates to substituted azadibenzocyclooctyne compounds. These substituted azadibenzocyclooctyne compounds, which may also be referred to as substituted DIBAC analogues, comprise one or more substituents on the aryl rings. The invention therefore relates to a compound of Formula (**5**): 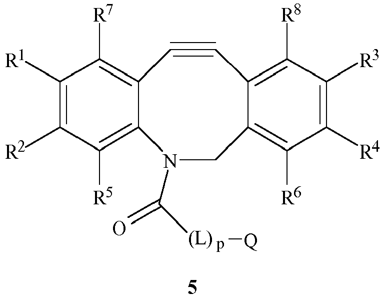 wherein:
R¹, R², R³, R⁴, R⁵, R⁶, R⁷ and R⁸ are independently selected from the group consisting of hydrogen, halogen, C₁- C₁₂ haloalkyl, -CN, -N₃, -NO₂, -NCX, -XCN, -N(R⁹)₂, -N⁺(R⁹)₃, -C(X)N(R⁹)₂, -C(X)R⁹, -C(X)XR⁹, -S(O)R⁹, -S(O)₂R⁹, -S(O)OR⁹, -S(O)₂OR⁹, -S(O)N(R⁹)₂, -S(O)₂N(R⁹)₂, -OS(O)R⁹, -OS(O)₂R⁹, -OS(O)OR⁹, -OS(O)₂OR⁹, -P(O)(R⁹)(OR⁹), -P(O)(OR⁹)₂, -OP(O)(OR⁹)₂, -XC(X)R⁹, -XC(X)XR⁹, -XC(X)N(R⁹)₂, -N(R⁹)C(X)R⁹, -N(R⁹)C(X)XR⁹ and -N(R⁹)C(X)N(R⁹)₂, wherein X is oxygen or sulfur and wherein R⁹ is independently selected from the group consisting of hydrogen, halogen, C₁ - C₂₄ alkyl groups, C₂ - C₂₄ (hetero)aryl groups, C₃ - C₂₄ alkyl(hetero)aryl groups and C₃ - C₂₄ (hetero)arylalkyl groups;
with the proviso that at least one of R¹, R², R³, R⁴, R⁵, R⁶, R⁷ and R⁸ is selected from the group consisting of halogen, C₁ - C₁₂ haloalkyl-CN, -N₃, -NO₂, -NCX, -XCN, -N(R⁹)₂, -N⁺(R⁹)₃, -C(X)N(R⁹)₂, -C(X)R⁹, -C(X)XR⁹, -S(O)R⁹, -S(O)₂R⁹, -S(O)OR⁹, -S(O)₂OR⁹, -S(O)N(R⁹)₂, -S(O)₂N(R⁹)₂, -OS(O)R⁹, -OS(O)₂R⁹, -OS(O)OR⁹, -OS(O)₂OR⁹, -P(O)(R⁹)(OR⁹), -P(O)(OR⁹)₂, -OP(O)(OR⁹)₂, -XC(X)R⁹, -XC(X)XR⁹, -XC(X)N(R⁹)₂, -N(R⁹)C(X)R⁹, -N(R⁹)C(X)XR⁹ and -N(R⁹)C(X)N(R⁹)₂, wherein X and R⁹ are as defined above;
p is 0 or 1;
L is a linking group selected from the group consisting of linear or branched C₁-C₂₀₀ alkylene groups, C₂-C₂₀₀ alkenylene groups, C₂-C₂₀₀ alkynylene groups, C₃-C₂₀₀ cycloalkylene groups, C₅-C₂₀₀ cycloalkenylene groups, C₈-C₂₀₀ cycloalkynylene groups, C₂-C₂₀₀ (hetero)arylene groups, C₃-C₂₀₀ alkyl(hetero)arylene groups, C₃-C₂₀₀ (hetero)arylalkylene groups, C₄-C₂₀₀ (hetero)arylalkenylene groups, C₅-C₂₀₀ (hetero)arylalkynylene groups, the alkylene groups, alkenylene groups, alkynylene groups, cycloalkylene groups, cycloalkenylene groups, cycloalkynylene groups, (hetero)arylene groups, alkyl(hetero)arylene groups, (hetero)arylalkylene groups, (hetero)arylalkenylene groups, (hetero)arylalkynylene groups optionally being substituted and/or optionally interrupted by one or more heteroatoms, preferably 1 to 100 heteroatoms, said heteroatoms preferably being selected from the group consisting of O, S, N and NR¹², wherein R¹² is independently selected from the group consisting of hydrogen, halogen, C₁ - C₂₄ alkyl groups, C₂ - C₂₄ (hetero)aryl groups, C₃ - C₂₄ alkyl(hetero)aryl groups and C₃ - C₂₄ (hetero)arylalkyl groups; and
Q is a functional group selected from the group consisting of hydrogen, halogen, R¹¹, -CH=C(R¹¹)₂, -C≡CR¹¹, -[C(R¹¹)₂C(R¹¹)₂O]_{q}-R¹¹ wherein q is in the range of 1 to 200, -CN, -N₃, -NCX, -XCN, -XR¹¹, -N(R¹¹)₂, -N⁺(R¹¹)₃, -C(X)N(R¹¹)₂, -C(R¹¹)₂XR¹¹, -C(X)R¹¹, -C(X)XR¹¹, -S(O)R¹¹, -S(O)₂R¹¹, -S(O)OR¹¹, -S(O)₂OR¹¹, -S(O)N(R¹¹)₂, -S(O)₂N(R¹¹)₂, -OS(O)R¹¹, -OS(O)₂R¹¹, -OS(O)OR¹¹, -OS(O)₂OR¹¹, -P(O)(R¹¹)(OR¹¹), -P(O)(OR¹¹)₂, -OP(O)(OR¹¹)₂, -Si(R¹¹)₃, -XC(X)R¹¹, -XC(X)XR¹¹, -XC(X)N(R¹¹)₂, -N(R¹¹)C(X)R¹¹, -N(R¹¹)C(X)XR¹¹ and -N(R¹¹)C(X)N(R¹¹)₂, wherein X is oxygen or sulfur and wherein R¹¹ is independently selected from the group consisting of hydrogen, halogen, C₁ - C₂₄ alkyl groups, C₂ - C₂₄ (hetero)aryl groups, C₃ - C₂₄ alkyl(hetero)aryl groups and C₃ - C₂₄ (hetero)arylalkyl groups.

In a preferred embodiment, X is O.

In another preferred embodiment, R⁹ is independently selected from the group consisting of hydrogen, halogen and C₁-C₁₂ alkyl groups, more preferably from the group consisting of hydrogen, halogen and C₁-C₆ alkyl groups, even more preferably from the group consisting of hydrogen, halogen and C₁ - C₄ alkyl groups. Most preferably, R⁹ is independently selected from the group consisting of hydrogen, F, Cl, Br, methyl, ethyl, propyl, *i*-propyl, butyl and *t*-butyl.

When R¹, R², R³, R⁴, R⁵, R⁶, R⁷ and/or R⁸ is a C₁ - C₁₂ haloalkyl group, the haloalkyl group is preferably selected from the group consisting of -CF₃, -CBr₃, -CCl₃, -CHBr₂, -CHCl₂, -CHF₂, -CH₂Br, -CH₂Cl and -CH₂F.

As specified above, at least one of R¹, R², R³, R⁴, R⁵, R⁶, R⁷ and R⁸ is selected from the group consisting of halogen, C₁ - C₁₂ haloalkyl-CN, -N₃, -NO₂, -NCX, -XCN, -N(R⁹)₂, -N⁺(R⁹)₃, -C(X)N(R⁹)₂, -C(X)R⁹, -C(X)XR⁹, -S(O)R⁹, -S(O)₂R⁹, -S(O)OR⁹, -S(O)₂OR⁹, -S(O)N(R⁹)₂, -S(O)₂N(R⁹)₂, -OS(O)R⁹, -OS(O)₂R⁹, -OS(O)OR⁹, -OS(O)₂OR⁹, -P(O)(R⁹)(OR⁹), -P(O)(OR⁹)₂, -OP(O)(OR⁹)₂, -XC(X)R⁹, -XC(X)XR⁹, -XC(X)N(R⁹)₂, -N(R⁹)C(X)R⁹, -N(R⁹)C(X)XR⁹ and -N(R⁹)C(X)N(R⁹)₂, wherein X and R⁹ are as defined above. The substituted DIBAC analogues according to the Formula (**5**) thus comprise one or more substituents. The term "substituent" in this context relates to a group that is present on an aryl moiety of the substituted DIBAC analogue, or an atom that is present on said aryl moiety wherein said atom is not a hydrogen atom. In other words, at least one of R¹, R², R³, R⁴, R⁵, R⁶, R⁷ and R⁸ in the compounds according to Formula (**5**) does not equal hydrogen. Preferably, the substituted DIBAC analogues according to the invention comprise 1, 2, 3 or 4, more preferably 1 or 2, substituents.

In a preferred embodiment, the one or more substituents present on the aryl groups of the substituted DIBAC analogues (**5**) are electron-withdrawing substituents having a positive value for the *para*-Hammett substituent constant σₚ and/or the *meta*-Hammett substituent σₘ. Groups with a positive value for σₚand/or σₘ include for example F, Cl, Br, I, NO₂, CN and many others. *para*-Hammett substituent constants σₚand *meta-*Hammett substituents σₘ are known for a large number of substituents (see for example C. Hansch et al., Chem. Rev. 1991, 91, 165 - 195, incorporated by reference). In a preferred embodiment, R¹, R², R³, R⁴, R⁵, R⁶, R⁷ and R⁸ are therefore independently selected from the group consisting of hydrogen and electron-withdrawing substituents having a positive value for the *para*-Hammett substituent constant σₚ and/or the *meta-*Hammett substituent σₘ, with the proviso that at least one of R¹, R², R³, R⁴, R⁵, R⁶, R⁷ and R⁸ is selected from the group consisting of electron-withdrawing substituents having a positive value for the *para*-Hammett substituent constant σₚand/or the *meta*-Hammett substituent σₘ. In other words, at least one of R¹, R², R³, R⁴, R⁵, R⁶, R⁷ and R⁸ is not hydrogen.

In another preferred embodiment, R¹, R², R³, R⁴, R⁵, R⁶, R⁷ and R⁸ are independently selected from the group consisting of hydrogen, halogen (preferably -F, -Cl, -Br), C₁ - C₁₂ haloalkyl, -CN, -NO₂, -NCO, -N⁺(R⁹)₃, -C(X)N(R⁹)₂, -C(X)R⁹, -C(X)XR⁹, -S(O)R⁹, -S(O)₂R⁹, -S(O)OR⁹, -S(O)₂OR⁹, -S(O)N(R⁹)₂, -S(O)₂N(R⁹)₂, -OS(O)₂R⁹, -XC(X)R⁹, -XC(X)XR⁹, -XC(X)N(R⁹)₂, wherein X and R⁹ are as defined above; with the proviso that at least one of R¹, R², R³, R⁴, R⁵, R⁶, R⁷ and R⁸ is selected from the group consisting of halogen (preferably -F, -Cl, -Br), C₁ - C₁₂ haloalkyl, -CN, -NO₂, -NCO, -N⁺(R⁹)₃, -C(X)N(R⁹)₂, -C(X)R⁹, -C(X)XR⁹, -S(O)R⁹, -S(O)₂R⁹, -S(O)OR⁹, -S(O)₂OR⁹, -S(O)N(R⁹)₂, -S(O)₂N(R⁹)₂, -OS(O)₂R⁹, -XC(X)R⁹, -XC(X)XR⁹, -XC(X)N(R⁹)₂, wherein X and R⁹ are as defined above.

In a more preferred embodiment, R¹, R², R³, R⁴, R⁵, R⁶, R⁷ and R⁸ are independently selected from the group consisting of hydrogen, halogen (preferably -F, -Cl, -Br), -CN, -NO₂, -N⁺(R⁹)₃, -C(O)N(R⁹)₂, -C(O)R⁹, -C(O)OR⁹, -S(O)R⁹, -S(O)₂R⁹, -S(O)OR⁹, -S(O)₂OR⁹, -S(O)N(R⁹)₂, -S(O)₂N(R⁹)₂ and -OS(O)R⁹, wherein R⁹ is as defined above; with the proviso that at least one of R¹, R², R³, R⁴, R⁵, R⁶, R⁷ and R⁸ is selected from the group consisting of halogen (preferably -F, -Cl, -Br), -CN, -NO₂, -N⁺(R⁹)₃, -C(O)N(R⁹)₂, -C(O)R⁹, -C(O)OR⁹, -S(O)R⁹, -S(O)₂R⁹, -S(O)OR⁹, -S(O)₂OR⁹, -S(O)N(R⁹)₂, -S(O)₂N(R⁹)₂ and -OS(O)R⁹, wherein R⁹ is as defined above.

In a further preferred embodiment, R¹, R², R³, R⁴, R⁵, R⁶, R⁷ and R⁸ are independently selected from the group consisting of hydrogen, halogen (preferably -F, -Cl, -Br), -CN, -NO₂, -N⁺(R⁹)₃, -C(O)R⁹, -C(O)OR⁹, -S(O)R⁹ and -S(O)₂R⁹, wherein R⁹ is as defined above; with the proviso that at least one of R¹, R², R³, R⁴, R⁵, R⁶, R⁷ and R⁸ is selected from the group consisting of -F, -Cl, -Br, -CN, -NO₂, -N⁺(R⁹)₃, -C(O)R⁹, -C(O)OR⁹, -S(O)R⁹ and -S(O)₂R⁹, wherein R⁹ is as defined above.

Even more preferably, R¹, R², R³, R⁴, R⁵, R⁶, R⁷ and R⁸ are independently selected from the group consisting of hydrogen, halogen (preferably -F, -Cl, -Br), -NO₂, -N⁺(R⁹)₃ and -CN; with the proviso that at least one of R¹, R², R³, R⁴, R⁵, R⁶, R⁷ and R⁸ is selected from the group consisting of halogen (preferably -F, -Cl, -Br), -NO₂, -N⁺(R⁹)₃ and -CN. Most preferably, R¹, R², R³, R⁴, R⁵, R⁶, R⁷ and R⁸ are independently selected from the group consisting of hydrogen, halogen (preferably -F, -Cl, -Br); with the proviso that at least one of R¹, R², R³, R⁴, R⁵, R⁶, R⁷ and R⁸ is selected from the group consisting of halogen (preferably -F, -Cl, -Br). As was already described above, also in these preferred embodiments at least one of R¹, R², R³, R⁴, R⁵, R⁶, R⁷ and R⁸ is not hydrogen.

In one specific embodiment, R⁷ and R⁸ are hydrogen. In another specific embodiment, R⁵ and R⁶ are hydrogen. In another specific embodiment, R⁵, R⁶, R⁷ and R⁸ are hydrogen.

In one embodiment, R¹ is equal to R³, and/or R² is equal to R⁴, and/or R⁷ is equal to R⁸, and/or R⁵ is equal to R⁶. In a preferred embodiment, R¹ is equal to R³, and R² is equal to R⁴, and R⁷ is equal to R⁸, and R⁵ is equal to R⁶.

In a preferred embodiment, R¹ and R³ are selected from the group consisting of F, Cl and Br, and it is further preferred that R¹ is equal to R³. More preferably, R² is equal to R⁴, and R⁷ is equal to R⁸, and R⁵ is equal to R⁶. Even more preferably, R², R⁴, R⁵, R⁶, R⁷ and R⁸ are all hydrogen. Yet even more preferably, R¹ is equal to R³; and R¹ and R³ are selected from the group consisting of F, Cl and Br; and R², R⁴, R⁵, R⁶, R⁷ and R⁸ are all hydrogen.

In another preferred embodiment, R² and R⁴ are selected from the group consisting of F, Cl and Br, and it is further preferred that R² is equal to R⁴. More preferably, R¹ is equal to R³, and R⁷ is equal to R⁸, and R⁵ is equal to R⁶. Even more preferably, R¹, R³, R⁵, R⁶, R⁷ and R⁸ are all hydrogen. Yet even more preferably, R² is equal to R⁴; and R² and R⁴ are selected from the group consisting of F, Cl and Br; and R¹, R³, R⁵, R⁶, R⁷ and R⁸ are all hydrogen.

In yet another preferred embodiment, R³ is selected from the group consisting of F, Cl and Br. In this embodiment it is preferred that R⁵ and R⁶ are hydrogen. It is also preferred that that R⁷ and R⁸ are hydrogen. More preferably, R¹, R² and R⁴ are hydrogen. The invention thus also relates to a compound according to Formula (**5a**), wherein R³ is selected from the group consisting of F, Cl and Br; R¹, R² and R⁴, R⁵, R⁶, R⁷ and R⁸ are hydrogen; and wherein L, p and Q are as defined above. In this embodiment, it is further preferred that R³ is Cl.

In yet another preferred embodiment, R⁴ is selected from the group consisting of F, Cl and Br. In this embodiment it is preferred that R⁵ and R⁶ are hydrogen. It is also preferred that R⁷ and R⁸ are hydrogen. More preferably, R¹, R² and R³ are hydrogen. The invention thus also relates to a compound according to Formula (**5c**), wherein R⁴ is selected from the group consisting of F, Cl and Br; R¹, R², R⁴, R⁵, R⁶, R⁷ and R⁸ are hydrogen; and wherein L, p and Q are as defined above. In this embodiment, it is further preferred that R⁴ is Br.

Substituted azadibenzocyclooctyne compounds (**5a**) and (**5c**) are shown below.

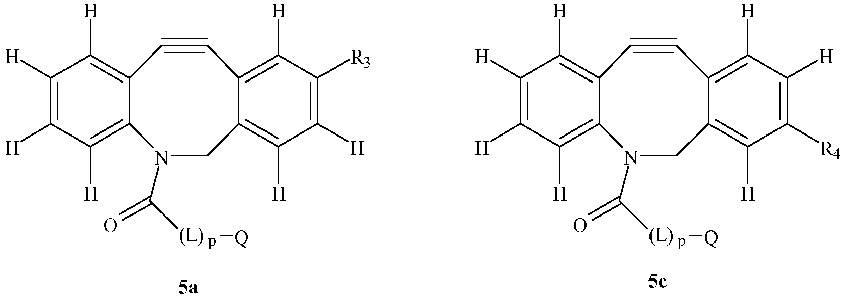

In one embodiment of the substituted azadibenzocyclooctyne compounds according the invention, p is 0, i.e. Q is bonded directly to the amide carbonyl group. In another embodiment, p is 1, in other words, Q is connected to the amide carbonyl group via linking unit L.

Linkers (L), also referred to as linking units or linking groups, are well known in the art. Examples of suitable linking units include (poly)ethylene glycol diamines (e.g. 1,8-diamino-3,6-dioxaoctane or equivalents comprising longer ethylene glycol chains), polyethylene glycol or polyethylene oxide chains, polypropylene glycol or polypropylene oxide chains and 1,x-diaminoalkanes wherein x is the number of carbon atoms in the alkane.

Another class of suitable linkers comprises cleavable linkers. Cleavable linkers are well known in the art. For example Shabat et al., Soft Matter 2012, 6, 1073, incorporated by reference herein, discloses cleavable linkers comprising self-immolative moieties that are released upon a biological trigger, e.g. an enzymatic cleavage or an oxidation event. Some examples of suitable cleavable linkers are peptide-linkers that are cleaved upon specific recognition by a protease, *e.g.* cathepsin, plasmin or metalloproteases, or glycoside-based linkers that are cleaved upon specific recognition by a glycosidase, *e.g.* glucoronidase, or nitroaromatics that are reduced in oxygen-poor, hypoxic areas

In a preferred embodiment of the substituted azadibenzocyclooctyne compounds according to the invention, the linking group L is selected from the group consisting of linear or branched C₁ - C₂₄ alkylene groups (preferably linear C₁ - C₂₄ alkylene groups), (poly)ethylene glycol diamines, polyethylene glycol chains, polyethylene oxide chains, polypropylene glycol chains, polypropylene oxide chains and 1,x-diaminoalkanes wherein x is the number of carbon atoms in the alkane and wherein x is in the range of 1 - 20. Said C₁ - C₂₄ alkylene group is preferablya C₁ - C₁₂ alkylene group, even more preferably a C₁ - C₈ alkylene group, yet even more preferably a C₁ - C₆ alkylene group, and most preferably a C₁ - C₄ alkylene group. Said C₁ - C₂₄ alkylene group may for example be a methylene, ethylene, propylene or butylene group.

Preferably, Q is selected from the group consisting of -CN, -N₃, -NCX, -XCN, -XR¹¹, -N(R¹¹)₂, -N⁺(R¹¹)₃, -C(X)N(R¹¹)₂, -C(R¹¹)₂XR¹¹, -C(X)R¹¹, -C(X)XR¹¹, -XC(X)R¹¹, -XC(X)XR¹¹, -XC(X)N(R¹¹)₂, -N(R¹¹)C(X)R¹¹, -N(R¹¹)C(X)XR¹¹ and -N(R¹¹)C(X)N(R¹¹)₂, wherein X and R¹¹ are as defined above. More preferably, X is oxygen. Most preferably, Q is selected from the group consisting of -OR¹¹, -SR¹¹, -N(R¹¹)₂, -N⁺(R¹¹)₃, -C(O)N(R¹¹)₂, -C(O)OR¹¹, -OC(O)R¹¹, -OC(O)OR¹¹, -OC(O)N(R¹¹)₂, -N(R¹¹)C(O)R¹¹, -N(R¹¹)C(O)OR¹¹ and -N(R¹¹)C(O)N(R¹¹)₂. Furthermore, the functional group Q may optionally be masked or protected. The R¹¹ groups may be selected independently from each other, which means that the two R¹¹ groups present in for example a -N(R¹¹)₂ substituent may be different from each other.

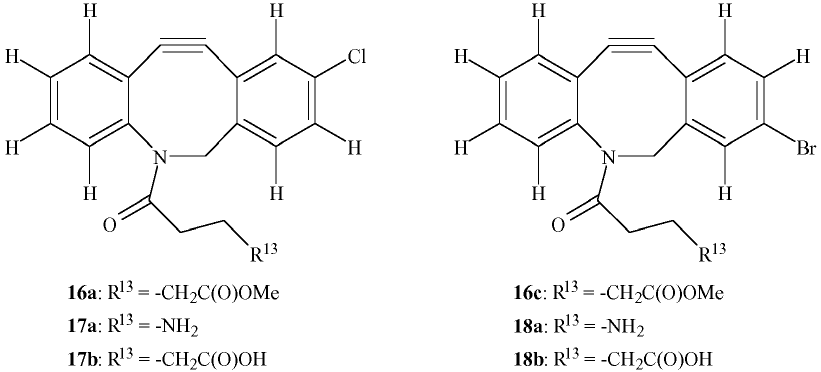

Specific examples of the substituted DIBAC analogues according to the invention include compounds of the Formula (**16a**), (**16c**), (**17a**), (**17b**), (**18a**) and (**18b**), as shown above.

### Synthesis of substituted azadibenzocyclooctyne compounds

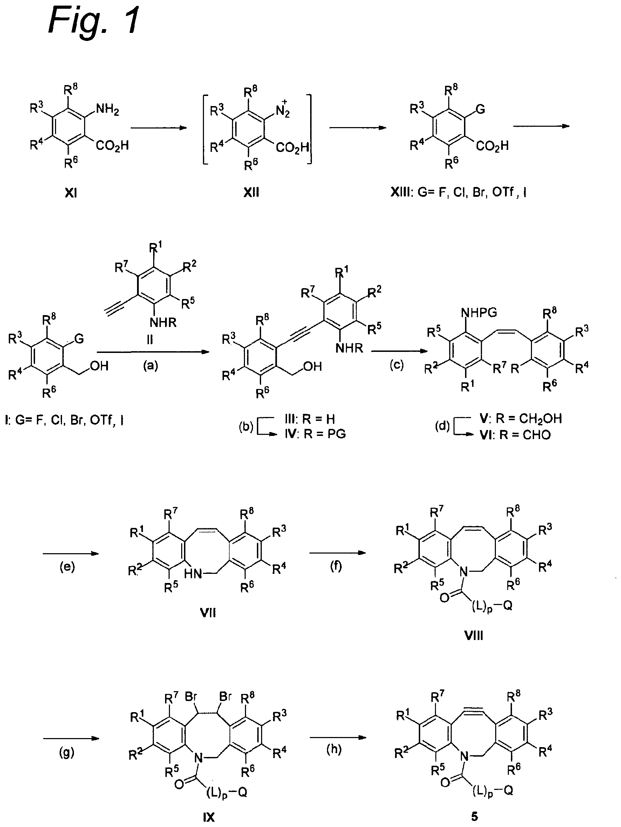

The substituted azadibenzocyclooctyne compounds according to the present invention are readily synthsized. A general route for the synthesis of the compounds of Formula (5) according to the invention is shown in Figure 1.

The invention thus also relates to a method for the manufacturing of a compound of the Formula (**5**), said method comprising the steps of:
(a) Sonogashira coupling of a substituted 2-G-benzyl alcohol (**I**), wherein G is selected from the group consisting of F, Cl, Br, I and OTf, preferably Cl, Br, I and OTf, with a substituted ortho-ethynylaniline (**II**) in order to obtain a compound of Formula (**III**);
(b) Protection of the amine hydrogen atom in (**III**) with a protecting group (PG) in order to obtain an alkyne of Formula (**IV**);
(c) Partial hydrogenation of the alkyne bond of (**IV**) in order to obtain an alkene of Formula (**V**);
(d) Oxidation of the benzyl alcohol moiety in (**V**) to an aldehyde moiety in order to obtain a compound of Formula (**VI**);
(e) Deprotection of the amine group, followed by ring-closing reductive amination in order to obtain an azadibenzocyclooctene (**VII**);
(f) Introduction of (L)ₚ-Q into azadibenzocyclooctene (**VII**) in order to obtain an azadibenzocyclooctene of Formula (**VIII**);
(g) Bromination of the alkene bond in azadibenzocyclooctene (**VIII**) in order to obtain a dibromo-azadibenzocyclooctene (**IX**); and
(h) Double elimination of dibromo-azadibenzocyclooctene (**IX**) in order to form a substituted azadibenzocyclooctyne of Formula (**5**) according to the invention;
wherein R¹, R², R³, R⁴, R⁵, R⁶, R⁷, R⁸, L, p and Q are as defined above.

Steps (a) - (h) of said method for the manufacturing of a compound of the Formula (**5**) are also shown in Scheme 2. Steps (a) - (h) are discussed in more detail below.

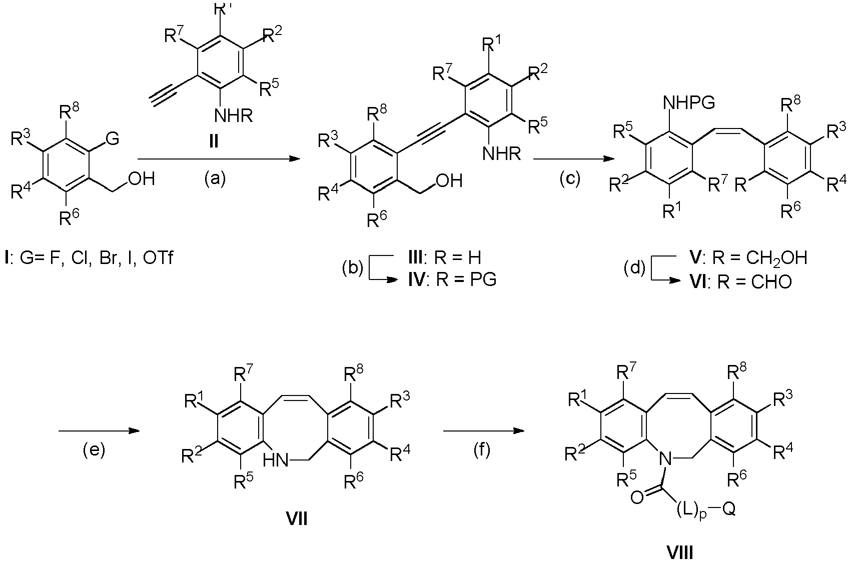

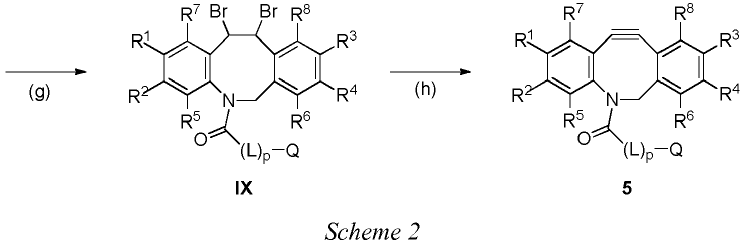

### Step (a): Sonogashira coupling of a substituted 2-G-benzyl alcohol (I) with a substituted ortho-ethynylaniline (II) in order to obtain a compound of Formula (III)

Step (a) is a Sonogashira coupling of a substituted 2-G-benzyl alcohol (**I**), wherein G is selected from the group consisting of F, Cl, Br, I and OTf (wherein OTf is triflate, i.e. trifluoromethanesulfonate), with a substituted ortho-ethynylaniline (**II**). In a preferred embodiment, G is Cl, Br, I or OTf, more preferably, Cl, Br or I and most preferably I.

Typical reaction conditions for a Sonogashira coupling are known to a person skilled in the art. Said coupling takes preferably place in the presence of a palladium catalyst (e.g. Pd(PPh₃)₂Cl₂), a copper catalyst (preferably a copper(I) halide, e.g. CuI) and a base (e.g. triethylamine). Said coupling is preferably executed with tetrahydrofuran (THF) as a solvent and under a mixed hydrogen-nitrogen atmosphere.

### Step (b): Protection of the amine hydrogen atom in (III) with a protecting group (PG) in order to obtain an alkyne of Formula (IV)

In step (b), the amine hydrogen atom in (**III**) is protected with a protecting group (PG). Protecting groups are known to a person skilled in the art, as are protecting groups that are suitable for the protection of an amine. Reaction conditions for the protection reaction strongly depend on the type of protecting group that is introduced, and are also known to the person skilled in the art.

In a preferred embodiment, the protecting group is selected from the group consisting of *t*-butyloxycarbonyl (BOC), fluorenylmethoxycarbonyl (Fmoc), benzyloxycarbonyl (Cbz), trichloroethyloxycarbonyl (Troc), nitrobenzenesulfonyl (Ns), and trifluoroacetyl (TFA).

### Step (c): Partial hydrogenation of the alkyne bond of (IV) in order to obtain an alkene of Formula (V)

The partial hydrogenation of the alkyne bond in (**IV**) in order to obtain alkene (**V**) is executed in the presence of a suitable hydrogenation catalyst, preferably a palladium catalyst, more preferably a palladium catalyst in the presence of a poisonous additive, preferably quinoline. Palladium hydrogenation catalysts are known in the art. Preferred palladium catalysts include Pd/BaSO₄ (e.g. 10%), Pd/CaCO₃ or Pd/C. Preferred reaction conditions strongly depend on the type of Pd-catalyst used in the hydrogenation, and are known to a person skilled in the art.

### Step (d): Oxidation of the benzyl alcohol moiety in (V) to an aldehyde moiety in order to obtain a compound of Formula (VI)

The oxidation of the primary alcohol in (**V**) in order to obtain aldehyde (**VI**) is executed by the use of suitable oxidant, preferably Dess-Martin periodinane in dichloromethane. Oxidants for the selective transformation of alcohols into aldehydes are known in the art. Preferred oxidants and/or oxidative conditions include Dess-Martin reagent, chromium-based reagents (PCC, PDC), Ley oxidation (Pr₄NRuO₄, NMO), Swern oxidant. Preferred reaction conditions strongly depend on the starting material.

### Step (e): Deprotection of the amine group, followed by ring-closing reductive amination in order to obtain an azadibenzocyclooctene (VII)

In step (e), the protective group on the amine hydrogen atom in (**VI**) is removed. Reaction conditions for deprotection strongly depend on the type of protecting group that is introduced, and are also known to the person skilled in the art.

Upon deprotection of the amine, a spontaneous ring-closure takes place by attack of the liberated amine onto the aldehyde and expulsion of water, thereby generating a cyclic imine.

The resulting imine may be *in situ* reduced to an amine (**VII**) by addition of a suitable reductive agent or after a work-up procedure, for example to neutralize the reagents applied to remove the protective group. Reductive methods for the selective reduction of imines into aldehydes are known in the art. Preferred reductive conditions include H₂/Pd-C, NaBH₄, LiBH₄, NaCNBH₃ in the presence of HOAc, Na(*t*BuO)₃BH, LiEt₃BH, Li(AcO)₃BH. Preferred reaction conditions strongly depend on the starting material.

### Step (f): Introduction of (L)ₚ-Q into azadibenzocyclooctene (VII) in order to obtain an azadibenzocyclooctene of Formula (VIII)

The generated amine (**VII**) may be acylated by a suitable electrophilic reagent in order to convert the amine into an amide functionality (as in compound **VIII**) and thereby at the same time introducing one (or more) functionalities at the other side of the chain. Methods for the acylation of amines are known in the art. Preferred reagents include acid anhydrides (symmetrical or mixed) acid halogenides (F, Cl, Br, I), acid in the presence of an activating reagents (DCC, EDC, BOP, PyBOP, T3P, HATU or the like). Suitable chains involve alkyl chains, aryl chains, oligomers of ethyleneglycol or ethylenediamine or the like, which may or may not be substituted. The functionality at the other side of the chain may involve a suitable reactive group (Q) for follow-up conjugation reactions, for example a carboxylic acid, an amine, an alcohol, a thiol. Such reactive groups are known in the art and are typically introduced in protected form in order to avoid undesired side-reactions during acylation of the amine or during subsequent transformations. Suitable protective groups for the functionalities mentioned are known to a person skilled in the art. The functionality at the other side of the chain may alternatively already contain the desired property, i.e. a "label", for example a reporter molecule for detection (radionuclide, fluorophore, NMR contrast agent, chelating moiety such as DTPA, DOTA, NOTA, a stable radical) or a solid phase. The term "label" is described in more detail below. Suitable reporter molecules are known in the art. Multiple such functional moieties may be present in a single linker.

### Step (g): Bromination of the alkene bond in azadibenzocyclooctene (VIII) in order to obtain a dibromo-azadibenzocyclooctene (IX)

The bromination of the alkene bond in (**VIII**) in order to obtain dibromide (**IX**) is executed in the presence of elemental bromine, preferably in dichloromethane.

### Step (h): Double elimination of the bromides in dibromo-azadibenzocyclooctene (IX) in order to form a substituted azadibenzocyclooctyne of Formula (5).

Elimination of the cyclooctene bromine atoms in dibromide (**IX**) in order to obtain the cyclic alkyne (**5**) is executed by treatment with excess of a suitable strong base, preferably KO*t*Bu in THF. Bases suitable for elimination of bromide are known to a person skilled in the art. Preferred bases include KO*t*Bu, NaO*t*Bu, LiHMDS, KHMDS, NaHMDS, LDA, KH, NaH. Preferred solvents for such elimination involve THF, Et₂O, dioxane, DMF or DMSO. Preferred reaction conditions strongly depend on the type of base used and the ease of elimination and may be performed with varying stoichiometries of base (from 2 up to >10 equivalents) and temperature (from -78 C to reflux).

As is shown in Scheme 3, substituted 2-G-benzyl alcohols (**I**), wherein G is selected from the group consisting of F, Cl, Br, I and OTf, more preferably Cl, Br, I and OTf, may be prepared starting from the corresponding anthranilic acid derivative (**XI**) via diazonium salt (**XII**) formation, followed by substitution with G (wherein G is selected from the group consisting of F, Cl, Br, I and OTf) in order to obtain (**XIII**). Subsequent reduction of the acids (**XIII**) provides the substituted 2-G-benzyl alcohols (**I**).

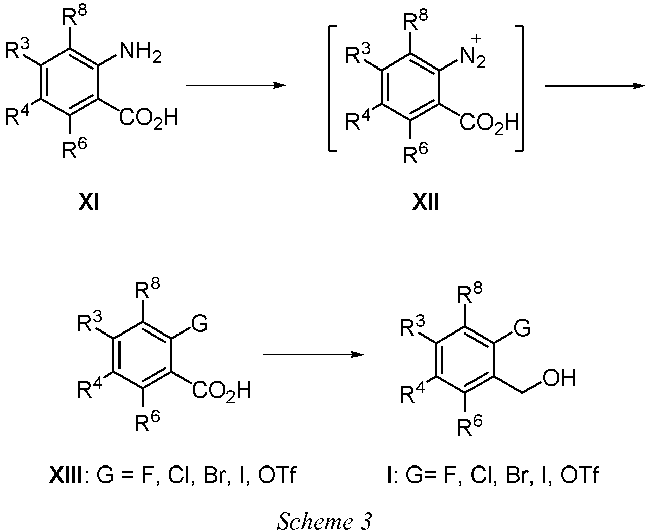

### Conjugates

The substituted azadibenzocyclooctyne compounds according to the present invention are very suitable for use in metal-free click reactions, and consequently these compounds are versatile tools in applications such as for example bioorthogonal labeling, imaging and/or modification, including surface modification, of a large range of target molecules. The present invention therefore also relates to a conjugate wherein a substituted azadibenzocyclooctyne compound according to the invention is conjugated to a label via a functional group Q.

The term "label" refers to any tag (including identifying tags or reporter molecules) that may be conjugated to a compound of the Formula (5). A wide variety of labels are known in the art, for a wide variety of different applications. Depending on the specific application, a suitable label for that specific application may be selected. Suitable labels for specific applications are known to the person skilled in the art, and include e.g. all kinds of fluorophores, biotin, polyethylene glycol (PEG) chains, polypropylene glycol (PPG) chains, mixed polyethylene/polypropylene glycol chains, radioactive isotopes, steroids, pharmaceutical compounds, lipids, amino acids, peptides, polypeptides (proteins), glycans (including oligo- and polysaccharides), nucleotides (including oligo- and polynucleotides), peptide tags and solid phases. Examples of suitable fluorophores are for example all kinds of Alexa Fluor (*e.g.* Alexa Fluor 555), cyanine dyes (*e.g.* Cy3 or Cy5), coumarin derivatives, fluorescein, rhodamine, allophycocyanin, chromomycin, and so on. Examples of suitable peptide tags include FLAG or HIS tags. Examples of suitable radioactive isotopes include ¹⁸F, ¹²⁶I, ⁶⁴Cu, ¹¹¹In, ⁹⁹Tc and ⁶⁸Ga. An example of a suitable glycan is concanavalin.

In a preferred embodiment, the label is selected from the group consisting of fluorophores, biotin, polyethylene glycol chains, polypropylene glycol chains, mixed polyethylene/polypropylene glycol chains, metal chelator complexes (with or without enclosed (radioactive) metal), radioactive isotopes, steroids, pharmaceutical compounds, lipids, amino acids, peptides, polypeptides, glycans, nucleotides, peptide tags and solid phases. A peptide is herein defined as a chain of amino acid monomers linked by peptide bonds, said chain comprising 2 to 50 amino acid monomers. A polypeptide is herein defined as a chain of amino acid monomers linked by peptide bonds, said chain comprising 51 or more amino acid monomers.

Functional group Q may be connected to the label directly, or indirectly via a linker or linking unit. Linking units are well know in the art. Linkers may e.g. have the general structure Q-L-Q, wherein Q and L are as defined above. Examples of suitable linking groups L are described above.

The present invention further relates to the use of a conjugate according to the invention for bioorthogonal labeling, imaging or modification of a target molecule.

### Modification of target molecules

The conjugates according to the present invention are successfully applied in bioorthogonal labeling, imaging or modification, including surface modification, of target molecules such as e.g. amino acids, peptides, polypeptides (i.e. proteins), lipids and glycans.

The present invention therefore also relates to a method for the modification of a target molecule, wherein a conjugate according to the present invention is reacted with a compound comprising a 1,3-dipole or a 1,3-(hetero)diene. As an example, the strain-promoted cycloaddition of a cycloalkyne with an azide (SPAAC) or with a nitrone (SPANC) was depicted in Scheme 1. The reaction of a cyclooctyne with a 1,3-(hetero)diene is known as a (hetero) Diels-Alder reaction. These reactions are also referred to as metal-free click reactions.

1,3-Dipolar compounds are well known in the art (cf. for example F.A. Carey and R.J. Sundberg, Advanced Organic Chemistry, Part A: Structure and Mechanisms, 3rd Ed., 1990, p. 635 - 637), and include nitrile oxides, azides, diazomethane, nitrones, nitrilamines, etc. Preferably, the compound comprising a 1,3-dipole is an azide-comprising compound, a nitrone-comprising compound or a nitrile oxide-comprising compound.

(Hetero) Diels-Alder reactions and 1,3-(hetero)dienes are also well known in the state of the art. Examples of 1,3-dienes include, amongst others, 1,3-butadiene, 1,3-cyclopentadiene, 1,3-cyclohexadiene, furan, pyrrole, and their substituted varieties. Examples of 1,3-heterodienes include amongst others 1-oxa-1,3-butadiene, 1-aza-1,3-butadiene, 2-aza-1,3-butadiene, 3-aza-1,3-butadiene, and their substituted varieties.

A large variety of target molecules, *i.e.* compounds comprising a 1,3-dipole or a 1,3-(hetero)diene, may be modified by the method according to the invention. Suitable target molecules are well known in the art and include, but are not limited to, biomolecules such as for example amino acids, proteins, peptides, glycans, lipids, nucleic acids, enzymes and hormones. In principle, any compound comprising a 1,3-dipole or a 1,3-(hetero)diene may be suitable as a target molecule.

Applications of the method for the modification of target molecules according to the present invention include diagnostic and therapeutic applications, cell labeling of living cells, for example MV3 cells, fibroblasts, Jurkat, CHO or HEK cells, modification of biopolymers (proteins, lipids, nucleic acids, glycans), enrichment of proteins and glycans for mass spectrometric analysis, tuning of polymer properties, surface modifications *etc.*

In one embodiment, the reaction of the conjugate is performed *in vitro.* In a preferred embodiment, the reaction is performed *in vivo*, *i.e.* under physiological conditions.

The conjugates according to the present invention that are applied in the modification of a target molecule are described above in great detail. One of the large advantages of these conjugates is that they may be applied both *in vivo* and *in vitro.* In addition, the here described conjugates suffer less from undesired aspecific lipophilic interactions, and show good reaction kinetics in metal-free click reactions. Another advantage is that the here described conjugates are easily synthesized and amenable to simple and straightforward modification of various parts of the conjugate. This makes it possible to "fine tune" a conjugate for a specific application, and optimise reaction kinetics for this application.

In a preferred embodiment, a compound of the Formula (**5**), or a conjugate thereof as described above, is reacted with a compound comprising a 1,3-dipole or a 1,3-(hetero)diene. Said reaction proceeds cleanly and rapidly, with excellent rate constants. For example the cycloaddition of (**16a**) or (**16c**) with benzyl azide in deuterated methabol proceeds rapidly and cleanly to the corresponding triazole adducts with excellent reaction kinetics (*k* = 0.9 M⁻¹ s⁻¹ for C1-DIBAC (**16a**) and 0.8 for Br-DIBAC (**16c**)).
C1-DIBAC (**16a**) and Br-DIBAC (**16c**) show an excellent rate constant comparable to BARAC (**2**), which is the fastest non-substituted benzannulated cyclooctyne currently known. The increase in rate as observed for the DIBAC analogues with electron-withdrawing groups C1-DIBAC (**16a**) and Br-DIBAC (**16c**) is relatively high, with an over two-fold rate enhancement. The reactivity of the azadibenzocyclooctyne compounds according to the invention may be modulated by the presence of the substituents.

Interestingly, substituents on DIBAC appear to have a larger effect on the rate than observed for BARAC. For example, for BARAC a fluoride at the *meta*-position relative to the alkyne results in a 1.1 to 1.3-fold rate enhancement, for DIBAC a chloride, which is less electron-withdrawing than fluoride, results in a more than two-fold rate increase. The same pattern is observed for substituents at the *para*-position.

### Pharmaceutical composition

The invention further relates to a composition comprising a conjugate according to the invention, further comprising a pharmaceutically acceptable carrier. Conjugates according to the invention are described in detail above. A wide variety of suitable pharmaceutically acceptable carriers are known in the art (cf. for example R.C. Rowe, P.J. Sheskey and P.J. Weller (Eds.), Handbook of Pharmaceutical Excipients, 4th Ed. 2003).

### Examples

### General experimental

Unless stated otherwise all chemicals were obtained from commercial sources and used without further purification. 1 M KO*^{t}*Bu in THF solution was purchased from Sigma-Aldrich. If no further details are given the reaction was performed under ambient atmosphere and temperature. Analytical thin layer chromatography (TLC) was performed on silica gel-coated plates (*Merck* 60 F254) with the indicated solvent mixture, visualization was done using ultraviolet (UV) irradiation (λ = 254 nm) and/or staining with KMnO₄. Purification by column chromatography was carried out using *Silicycle* silica gel (0.040-0.063 mm, and ca. 6 nm pore diameter). THF and CH₂Cl₂ were dried over an activated alumina column using an MBraun SPS800 solvent purification system. NEt₃ was distilled under N₂-atmosphere from CaH₂.
**Infrared (IR) Spectroscopy:** IR spectra were recorded on an ATI Matson Genesis Series FTIR spectrometer fitted with an ATR cell. The vibrations (v) are given in cm⁻¹.
**Nuclear Magnetic Resonance (NMR) Spectroscopy:** ¹H-NMR spectra were recorded on a *Varian Inova 400* (400 MHz) for room temperature measurements and a *Varian Inova 500* (500 MHz) for low temperature measurements. ¹³C-NMR spectra were recorded on a *Bruker DMX300* (75 MHz) spectrometer. Unless stated otherwise all spectra were taken at ambient temperature. ¹H-NMR chemical shifts (δ) are reported in parts per million (ppm) relative to a residual proton peak of the solvent, δ = 3.31 for CD₃OD and δ = 7.26 for CDCl₃. Broad peaks are indicated by the addition of br. Coupling constants are reported as a *J*-value in Hertz (Hz). In case of rotamers the spectrum was taken at lower temperature to freeze the compound in its two rotamer states, causing separate peaks for each rotamer. In these cases shifts, coupling constants and integrals are given of each separate peak. ¹³C-NMR chemical shifts (δ) are reported in ppm relative to CD₃OD (δ = 49.0) or CDCl₃ (δ = 77.0). If rotamers are observed in the spectrum, the minor rotamer peaks are labeled with *.
**Mass Spectrometry (MS):** High Resolution Mass Analyses were performed using Electrospray Ionization on a JEOL AccuToF.

### Synthesis

### Example 1: 5-Chloro-2-iodobenzoic acid (7a)

2-Amino-4-chlorobenzoic acid (**6a**, 10.0 g, 58.2 mmol) was dissolved in DMSO (100 mL), and 30% H₂SO₄ was added (100 mL). The solution was cooled to 0 °C, whereupon NaNO₂ (8.8 g, 129 mmol) was added. The reaction was stirred for two hours at room temperature, after which a solution of KI (19.3 g, 106 mmol) in H₂O (50 mL) was added. After one hour, an additional portion of KI (9.7 g, 58.2 mmol) in H₂O (25 mL) was added. In addition, DMSO (50 mL) was added to keep the reaction mixture solubilized. After one additional hour, EtOAc (300 mL) was added, and the organic layer was washed with H₂O (3 × 200 mL) and brine (200 mL), and subsequently dried over MgSO₄. The solvents were removed in vacuo to obtain crude **7a** as white solid. **7a** was not further purified and used as a crude in the following reaction. ¹H-NMR (400 MHz, CD₃OD) δ: 8.01 (d, *J* = 2.1 Hz, 1H), 7.76 (d, *J =* 8.4 Hz, 1H), 7.45 (dd, *J* = 8.4, 2.1 Hz, 1H). ¹³C-NMR (75 MHz, CD₃OD) δ: 168.9, 141.7, 138.6, 136.0, 132.7, 129.3, 95.0. HRMS (EI+) *m*/*z* calcd for C₇H₄O₂ClI [M]^{•+} 281.8945, found 281.8936.

### Example 2: 2-Amino-4-bromo-5-chlorobenzoic acid (6b)

2-Amino-4-chlorobenzoic acid (1.1 g, 6.4 mmol) was dissolved in acetic acid (8 mL) and Br₂ (0.33 mL, 6.4 mmol) was added. The mixture was stirred at room temperature for 4 hours and subsequently poured into saturated aqueous NaHSO₃ (50 mL). The H₂O-layer was extracted with EtOAc (2 × 50 mL), and the combined organic layers were washed with water (2 × 50 mL), brine (50 mL), and subsequently dried over MgSO₄. The solvents were evaporated under reduced pressure to obtain **6b** as a mixture of two products. **6b** was not further purified and used as a crude in the following reaction. ¹H-NMR (400 MHz, CD₃OD) δ: 8.00 (s, 1H), 6.92 (s, 1H).

### Example 3: 4-Bromo-5-chloro-2-iodobenzoic acid (7b)

Crude 2-amino-5-bromo-4-chlorobenzoic acid **6b** (6.0 g, 24 mmol) was dissolved in DMSO (100 mL) and 30% H₂SO₄ (100 mL) and the resulting mixture was cooled to 0 °C. NaNO₂ (3.6 g, 53 mmol) was added and the mixture was stirred for 2 hours at room temperature. After this time, a solution of KI (8.0 g, 48 mmol) in H₂O (40 mL) was added. After one hour an additional portion of KI (4.0 g, 24 mmol) in H₂O (20 mL) was added. After one more hour, EtOAc (200 mL) was added, and the organic layer was washed with H₂O (2 × 200 mL) and brine (200 mL), and dried over MgSO₄. The solvents were removed in vacuo to obtain **7b** as a mixture of two products. **7b** was used as a crude in the following reaction. ¹H-NMR (400 MHz, CD₃OD) δ: 8.16 (s, 1H), 8.07 (s, 1H).

### Example 4: 5-Bromo-2-iodobenzoic acid (7c)

2-Amino-5-bromobenzoic acid (**6c**, 2.0 g, 9.2 mmol) was dissolved in DMSO (50 mL) and 30% H₂SO₄ (50 mL) and NaNO₂ (0.89 g, 13 mmol) were added. The reaction mixture was stirred for 1 hour at room temperature, whereupon a solution of KI (3.1 g, 19 mmol) in H₂O (20 mL) was added and the reaction mixture was stirred for another hour. Next, another portion of KI (3.1 g, 19 mmol) in H₂O (10 mL) was added and the reaction mixture was stirred for an additional hour. The reaction mixture was quenched with a saturated aqueous Na₂SO₃-solution (75 mL), EtOAc (100 mL) was added and the layers were separated. Hereupon, the H₂O-layer was extracted with EtOAc (100 mL). The combined organic layers were washed with H₂O (2 × 100 mL) and brine (100 mL). The organic layer was dried over MgSO₄ and concentrated *in vacuo* to afford compound 7c as a yellow solid. 7c was not further purified further and used as a crude in the following reaction. *R*_{F} = 0.05 (EtOAc/*n*-heptane, 1:4). ¹H-NMR (400 MHz, CDCl₃) δ: 8.13 (d, *J* = 2.4 Hz, 1H), 7.90 (d, *J* = 8.4 Hz, 1H), 7.33 (dd, *J =* 8.4, 2.4 Hz, 1H).

### Example 5: 2-Iodo-5-nitrobenzoic acid (7d)

2-Amino-5-nitrobenzoic acid (**6d**, 1.82 g, 10 mmol) was dissolved in DMSO (50 mL) and 30% H₂SO₄ (50 mL) was added. The resulting mixture was heated for two hours at 50 °C. The reaction was cooled to 0 °C and a solution of NaNO₂ (970 mg, 14 mmol) in water (25 mL) was added. The mixture was stirred at 0 °C for one hour, whereupon a solution of KI (5.0 g, 30 mmol) in H₂O (10 mL) was added and the mixture was stirred for 1 hour at room temperature. Next, another portion of KI (5 g, 30 mmol) in H₂O (10 mL) was added and the mixture was stirred for an additional hour. EtOAc (100 mL) was added and the reaction was quenched with saturated aqueous NaHSO₃ (100 mL). The organic layer was washed with water (2 × 100 mL) and brine (100 mL) and subsequently dried over MgSO₄. The solvents were evaporated under reduced pressure and the crude product was obtained as yellow solid (12.0 g, 120%). **7d** was not further purified and used as a crude in the following reaction. ¹H-NMR (400 MHz, CD₃OD) δ: 8.54 (d, *J =* 2.7 Hz, 1H), 8.29 (d, *J =* 8.6 Hz, 1H), 8.01 (dd, *J* = 8.7, 2.7 Hz, 1H). ¹³C-NMR (75 MHz, CD₃OD) δ: 168.0, 149.2, 144.1, 139.2, 127.1, 125.8, 103.0, 49.6, 49.3, 49.1, 48.8, 48.5. FT-IR vₘₐₓ (cm⁻¹): 2932, 1722, 1588, 151, 1342, 1295, 1022, 1234, 728. HRMS (EI+) *m*/*z* calcd for C₇H₄NO₄I [M]^{•+} 292.9185, found 292.9184.

### Example 6: (4-Chloro-2-iodophenyl)methanol (8a)

4-chloro-2-iodobenzoic acid **7a** (15 g, 53 mmol) was dissolved in dry THF (250 mL) and the solution was cooled to 0 °C. Hereupon, NEt₃ (11 mL, 80 mmol) and ethyl chloroformate (7.6 mL, 80 mmol) were added. The reaction was stirred for 1.5 hour and subsequently NaBH₄ (8.0 g, 210 mmol) was added in four portions. After 1.5 hour, additional NaBH₄ (4.0 g, 105 mmol) was added and the reaction was stirred for another hour. Hereupon, the reaction was quenched with H₂O (100 mL) and EtOAc (200 mL) was added. The organic layer was washed with H₂O (3 × 150 mL), brine (100 mL) and subsequently dried over MgSO₄. The solvents were removed under reduced pressure and the crude product was obtained by gradient column chromatography (EtOAc/*n*-heptane, 1:9 to 1:6). Compound **8a** was obtained as white solid (8.4 g, 75% over 2 steps). ¹H-NMR (400 MHz, CDCl₃) δ: 7.82 (s, 1H), 7.45-7.33 (m, 2H), 4.65 (d, *J* = 6.2 Hz, 2H), 1.94 (t, *J* = 6.2 Hz, 1H). ¹³C-NMR (75 MHz, CDCl₃) δ: 141.1, 138.3, 133.8, 128.8, 128.6, 96.9, 68.6. HRMS (EI+) *m*/*z* calcd for C₇H₆OClI [M]^{•+} 267.9152, found 267.9160.

### Example 7: (4-Bromo-5-chloro-2-iodophenyl)methanol (8b)

Crude 5-bromo-4-chloro-2-iodobenzoic acid (**7b**, 11 g, 30 mmol) was dissolved in dry THF (100 mL) and the solution was cooled to 0 °C. Next, NEt₃ (6.2 mL, 44 mmol) and ethyl chloroformate (4.3 mL, 44 mmol) were added. The reaction mixture was stirred for 1 hour and subsequently a solution of NaBH₄ (2.24 g, 59 mmol) in H₂O (10 mL) was added. The mixture was stirred another hour, prior to quenching with H₂O (100 mL). The H₂O-layer was extracted with EtOAc (2 × 100 mL), and the combined organic layers were washed with H₂O (2 × 150 mL) and brine (150 mL). The organic layer was dried over MgSO₄ and the solvents were removed under reduced pressure. The crude product was purified using column chromatography (EtOAc/*n*-heptane, 1:6) to obtain **7b** as a white solid (3.84 g, 38% over 3 steps). ¹H-NMR (400 MHz, CDCl₃) δ: 7.88 (s, 1H), 7.72 (s, 1H), 4.62 (dd, *J* = 6.0, 0.7 Hz, 2H), 1.96 (t, *J* = 6.1 Hz, 1H). ¹³C-NMR (75 MHz, CDCl₃) δ: 142.8, 139.5, 139.2, 135.7, 132.4, 122.9, 94.3, 68.0, 52.4.

### Example 8: (5-Bromo-2-iodophenyl)methanol (8c)

5-Bromo-2-iodobenzoic acid (7c) (750 mg, 2.3 mmol) was dissolved in dry THF (25 mL) and the reaction mixture was cooled to 0 °C. NEt₃ (0.48 mL, 3.4 mmol) and ethyl chloroformate (0.33 mL, 3.4 mmol) were added and the reaction mixture was stirred for 1 hour. Next a solution of NaBH₄ (130 mg, 3.4 mmol) in H₂O (2 mL) was added and the mixture was stirred for 1.5 hour. The reaction was quenched with H₂O (15 mL), whereupon CH₂Cl₂ (20 mL) was added and the layers were separated. Hereupon, the H₂O-layer was extracted with CH₂Cl₂ (20 mL). Subsequently, the combined organic layers were washed with H₂O (25 mL) and brine (25 mL), dried over MgSO₄ and *concentrated in vacuo.* The crude product was purified by gradient column chromatography (*n*-heptane/EtOAc, 19:1 to 9:1) to obtain compound 7c as a white solid (410 mg, 54% over 2 steps). *R*_{F} = 0.40 (EtOAc/*n*-heptane, 1:4). ¹H-NMR (400 MHz, CDCl₃) δ: 7.65 (d, *J* = 8.3 Hz, 1H), 7.63 (d, *J* = 2.4 Hz, 1H), 7.14 (dd, *J* = 8.3, 2.5 Hz, 1H), 4.64 (d, *J* = 6.1 Hz, 2H), 1.96 (t, *J* = 6.2 Hz, 1H).

### Example 9: (2-Iodo-5-nitrophenyl)methanol (8d)

2-Iodo-5-nitrobenzoic acid (**7d**) (3.0 g, 10.2 mmol) was dissolved in dry THF (100 mL) and the reaction was cooled to 0 °C. NEt₃ (2.1 mL, 15.4 mmol) and ethyl chloroformate (1.5 mL, 15.4 mmol) were added and the reaction was stirred for 1 hour. Next, a solution of NaBH₄ (0.78 g, 20.5 mmol) in H₂O (5 mL) was added and the reaction was stirred for 1.5 hour. After this time, an additional portion of NaBH₄ (0.78 g, 20.5 mmol) in H₂O (5 mL) was added and the reaction was stirred for an additional 30 minutes. The reaction was then quenched by the addition of H₂O (20 mL). The reaction was diluted with EtOAc (150 mL) and the organic layer was washed with H₂O (2 × 100 mL) and brine (100 mL) and subsequently dried over MgSO₄. The solvents were *removed in vacuo* and the crude product was purified by gradient column chromatography (EtOAc/*n*-heptane, 1:9 to 1:3). Compound **8d** was obtained as an orange solid (1.32 g, 55% over 2 steps). ¹H-NMR (400 MHz, CDCl₃) δ: 8.36 (d, *J =* 2.8 Hz, 1H), 8.01 (d, *J =* 8.5 Hz, 1H), 7.85 (dd, *J =* 8.6, 2.8 Hz, 1H), 4.75 (d, *J* = 3.4 Hz, 2H), 2.10 (t, *J* = 5.0 Hz, 1H). HRMS (EI+) *m*/*z* calcd for C₇H₆NO₃I [M]^{•+} 278.9393, found 278.9396.

### Example 10: (2-((2-Aminophenyl)ethynyl)-5-chlorophenyl)methanol (9a)

Compound **8a** (8.5 g, 29.9 mmol), Pd(PPh₃)₂Cl₂ (430 mg, 0.60 mmol), and CuI (57 mg, 0.30 mmol) were added to a flame-dried flask. The flask was evacuated and refilled with an N₂/H₂-mixture (3:2) three times. THF (150 mL) and NEt₃ (12.4 mL, 89 mmol) were bubbled through with an N₂/H₂-mixture (3/2) for 10 minutes and subsequently added to the reaction mixture. After this time, 2-ethynylaniline (3.75 mL, 33 mmol) was added, and the mixture was stirred overnight under N₂/H₂-atmosphere.The reaction mixture was diluted with CH₂Cl₂ (250 mL) and the organic layer was washed with H₂O (3 × 250 mL). The H₂O-layers were combined and back-extracted with CH₂Cl₂ (150 mL). The organic layers were combined and washed with brine (150 mL). The solvents were removed under reduced pressure and the crude product was purified by gradient column chromatography (EtOAc/*n*-heptane, 1:4 to 1:1) to obtain **9a** as a yellow solid (7.3 g, 95%). ¹H-NMR (400 MHz, CDCl₃) δ: 7.52 (d, *J* = 1.9 Hz, 1H), 7.35 (t, *J* = 8.7 Hz, 2H), 7.29 (dd, *J* = 8.2, 2.1 Hz, 1H), 7.19-7.14 (m, 1H), 6.74-6.70 (m, 2H), 4.83 (d, *J=* 4.8 Hz, 2H), 4.41 (br s, 2H), 2.06 (t, *J=* 5.6 Hz, 1H). ¹³C-NMR (75 MHz, CDCl₃) δ: 148.34, 140.24, 133.36, 132.08, 131.58, 130.35, 128.92, 128.47, 123.70, 117.92, 114.53, 107.03, 92.26, 90.84, 63.69. HRMS (ESI+) *m*/*z* calcd for C₁₅H₁₃ClNO [M+H]⁺ 258.0686, found 258.0677.

### Example 11: (2-((2-Aminophenyl)ethynyl)-5-bromo-4-chlorophenyl)methanol (9b)

Compound **8b** (3 g, 8.6 mmol), Pd(PPh₃)₂Cl₂ (0.121 g, 0.17 mmol) and CuI (0.016 g, 0.086 mmol) were added to a flame-dried Schlenk flask. The flask was subsequently evacuated and refilled with an N₂/H₂-mixture (3:2) three times. At the same time, dry THF (150 mL) and dry NEt₃ (3.6 mL, 25.9 mmol) were bubbled with a N₂/H₂-mixture for 10 minutes. The bubbled solutions were subsequently added to the Schlenk flask. Next, 2-ethynylaniline (1.08 mL, 9.5 mmol) was added and the mixture was stirred for 4 hours under N₂/H₂-atmosphere. Hereupon, CH₂Cl₂ (150 mL) was added and the organic layer was washed with H₂O (3 × 100 mL). The H₂O-layers were combined and back-extracted with CH₂Cl₂ (150 mL). The organic layers were combined and dried over MgSO₄. The solvents were removed under reduced pressure and the crude product was purified by gradient column chromatography (EtOAc/*n*-heptane, 1:4 to 1:2). Compound **9b** was obtained as a white solid (2.74 g, 94%). ¹H-NMR (400 MHz, CDCl₃) δ 7.78-7.69 (m, 1H), 7.60 (s, 1H), 7.33 (dd, *J* = 8.0, 1.6 Hz, 1H), 7.18 (ddd, *J* = 8.2, 7.4, 1.6 Hz, 1H), 6.80-6.57 (m, 2H), 4.83 (d, *J* = 5.7 Hz, 2H), 4.37 (br s, 2H), 2.01 (t, *J* = 6.2 Hz, 1H). ¹³C-NMR (75 MHz, CD₃OD) δ: 150.7, 144.3, 133.7 (2C), 133.2, 133.0, 131.4, 123.7, 122.9, 118.2, 115.7, 107.8, 94.5, 90.5, 62.7. HRMS (ESI+) *m*/*z* calcd for C₁₅H₁₂BrClNO [M+H]⁺ 335.9791, found 335.9781.

### Example 12: (2-((2-Aminophenyl)ethynyl-5-bromophenyl)methanol (9c)

(5-Bromo-2-iodophenyl)methanol (**8c**) (106 mg, 0.34 mmol), Pd(PPh₃)₂Cl₂ (7.0 mg, 0.01 mmol) and CuI (1.2 mg, 6.3 µmol) were added to a flame-dried Schlenk flask. The flask was evacuated and refilled with an N₂/H₂-mixture (3:2) three times. Dry THF (3 mL) and dry NEt₃ (71 µL, 0.51 mmol) were bubbled through with an N₂/H₂ mixture (3:2) for 10 minutes and subsequently added to the mixture. After this time, 2-ethynylaniline (0.060 mL, 0.58 mmol) was added and the mixture was stirred for 16 hours under an N₂/H₂-atmosphere. The reaction mixture was diluted with CH₂Cl₂ (5 mL) and the organic layer was washed with H₂O (5 mL). The water layer was then back-extracted with CH₂Cl₂ (2 × 5 mL). The combined organic layers were washed with H₂O (15 mL) and brine (20 mL). Next, the organic layer was dried over MgSO₄ and *concentrated in vacuo.* The crude product was purified by gradient column chromatography (EtOAc/*n*-heptane, 1:6 to 1:2) to obtain **9c** as a yellow solid (102 mg, 100%). *R*_{F} = 0.40 (EtOAc/*n*-heptane, 1:2). ¹H-NMR (300 MHz, CD₃OD) δ: 7.68-7,67 (m, 1H), 7.43-7.42 (m, 2H), 7.28 (ddd, *J* = 7.7, 1.5, 0.4 Hz, 1H), 7.12 (ddd, *J* = 7.3, 6.6, 1.6 Hz, 1H) 6.79-6.76 (m, 1H) 6.64 (td, *J* = 7.7, 1.1 Hz, 1H), 4.80 (d, *J* = 0.6 Hz, 2H). ¹³C-NMR (75 MHz, CD₃OD) δ: 148.6, 144.2, 132.4, 131.0, 129.4, 129.2, 129.1, 121.4, 120.0, 116.5, 113.8, 106.5, 91.6, 89.8, 61.3. FT-IR vₘₐₓ film (cm⁻¹): 3360, 2923, 2850, 2362, 2202, 1610, 1489, 1450, 815, 750. HRMS (ESI+) *m*/*z* calcd for C₁₅H_{113b}rNO [M+H]⁺ 302.0181, found 302.0169.

### Example 13: (2-((2-Aminophenyl)ethynyl)-5-nitrophenyl)methanol (9d)

(2-Iodo-5-nitrophenyl)methanol (**9d**) (1.32 g, 4.73 mmol), Pd(PPh₃)₂Cl₂ (66 mg, 0.095 mmol) and CuI (5 mg, 0.047 mmol) were added to a flame-dried flask. The flask was evacuated and refilled with an N₂/H₂-mixture (3/2). Dry THF (70 mL) and dry NEt₃ (2.0 mL, 14 mmol) were bubbled through with an N₂/H₂ mixture (3:2) for 10 minutes and subsequently added to the mixture. Hereupon, 2-ethynylaniline (0.81 mL, 7.1 mmol) was added, and the mixture was stirred for 3 hours.The reaction mixture was diluted with CH₂Cl₂ (100 mL) and washed with H₂O (3 × 100 mL). The H₂O-layers were combined and back-extracted with CH₂Cl₂ (100 mL). The organic layers were combined and dried over MgSO₄. The solvents were removed under reduced pressure and the crude product was purified by gradient column chromatography (EtOAc/*n*-heptane, 1:4 to 2:1). Compound **9d** was obtained as a red solid (1.13 g, 89 % yield). ¹H-NMR (400 MHz, CDCl₃) δ: 8.38 (d, *J* = 2.0 Hz, 1H), 8.16 (dd, *J* = 8.5, 2.0 Hz, 1H), 7.67 (d, *J* = 8.5 Hz, 1H), 7.50-7.32 (m, 1H), 7.24-7.16 (m, 1H), 6.87-6.53 (m, 2H), 4.98 (d, *J=* 4.0 Hz, 2H), 4.44 (br s, 2H), 2.10 (t, *J=* 6.1 Hz, 1H). ¹³C-NMR (75 MHz, CDCl₃) δ: 177.2, 165.2, 143.4, 132.5, 132.3, 131.1, 128.5, 127.9, 122.6, 122.3, 118.1, 114.7, 96.7, 96.2, 63.4. HRMS (ESI+) *m*/*z* calcd for C₁₅H₁₃N₂O₃ [M+H]⁺ 269.0926, found 269.0916.

### Example 14: tert-Butyl (2-((5-chloro-2-(hydroxymethyl)phenyl)ethynyl)phenyl) carbamate (10a)

Compound **9a** (7.3 g, 28.4 mmol) was dissolved in THF (34 mL) and Boc₂O (7.4 g, 33.9 mmol) was added. The mixture was heated to 70 °C and stirred for three days. The mixture was diluted with EtOAc (300 mL) and the organic layer was washed with H₂O (3 × 200 mL), and brine (200 mL) and subsequently dried over MgSO₄. The solvents were removed under reduced pressure and the thus obtained crude product was purified by gradient column chromatography (EtOAc/*n*-heptane, 1:7 to 1:4) yielding **10a** as a white solid (8.24 g, 81%). ¹H-NMR (400 MHz, CDCl₃) δ: 8.13 (br d, *J =* 7.0 Hz, 1H), 7.83 (s, 1H), 7.56 (d, *J =* 2.0 Hz, 1H), 7.45 (dd, *J =* 7.7, 1.4 Hz, 1H), 7.39-7.31 (m, 3H), 7.01 (t, *J=* 7.3 Hz, 1H), 4.88 (d, *J* = 4.5 Hz, 2H), 2.38 (br s, 1H), 1.57 (s, 9H). ¹³C-NMR (75 MHz, CDCl₃) δ: 152.6, 140.2, 139.8, 133.6, 131.8, 131.5, 130.2, 129.5, 128.8, 123.6, 122.3, 118.1, 111.0, 92.6, 90.5, 81.3, 63.7, 28.3 (3C). HRMS (ESI+) *m*/*z* calcd [M+Na]⁺ for C₂₀H₂₀ClNNaO₃ 380.1029, found 380.1019.

### Example 15: tert-Butyl (2-((4-bromo-S-chloro-2-(hydroxymethyl)phenyl)ethynyl)-phenyl) carbamate (10b)

Compound **9b** (1.8 g, 5.35 mmol) was dissolved in THF (5.4 mL) and Boc₂O (1.17 g, 5.35 mmol) was added. The mixture was heated to 70 °C and stirred overnight. The reaction mixture was diluted with EtOAc (100 mL) and the organic layer was washed with H₂O (3 × 100 mL), and brine (100 mL), and was subsequently dried over MgSO₄. The solvents were removed under reduced pressure and the crude product was purified by gradient column chromatography (EtOAc/*n*-heptane, 1:8 to 1:2). Compound **10b** was obtained as a white solid (1.34 g, 57%), also starting material 9b was re-obtained (540 mg, 30%). ¹H-NMR (400 MHz, CDCl₃) δ: 8.12 (d, *J* = 5.8 Hz, 1H), 7.74 (br s, 1H), 7.72 (s, 1H), 7.63 (s, 1H), 7.44 (ddd, *J =* 7.7, 1.6, 0.5 Hz, 1H), 7.40 - 7.31 (m, 1H), 7.02 (dt, *J =* 7.6, 1.1 Hz, 1H), 4.85 (d, *J =* 4.8 Hz, 2H), 2.43 (br s, 1H), 1.57 (s, 9H). ¹³C-NMR (75 MHz, CDCl) δ: 151.7, 141.2, 140.2, 133.8, 133.2, 133.1, 131.5, 130.4, 122.9, 122.3, 118.2, 118.0, 110.7, 91.8, 91.3, 81.4, 63.1, 28.3 (3C). HRMS (ESI+) *m*/*z* calcd [M+H]⁺ for C₂₀H₂₀BrClNO₃ 436.0301, found 436.0315.

### Example 16: tert-Butyl 2-((4-bromo-2-hydroxymethyl)phenyl)ethynyl)phenyl)-carbamate (10c)

Compound 9c (381 mg, 1.26 mmol) was dissolved in THF (1.2 mL) and Boc₂O (275 mg, 1.26 mmol) was added. The reaction was stirred for two days at 70 °C in a sealed tube. The reaction mixture was diluted with CH₂Cl₂ (10 mL) and the organic layer was washed with H₂O (15 mL). The H₂O-layer was extracted with CH₂Cl₂ (10 mL). The combined organic layers were washed with H₂O (2 × 10 mL) and brine (10 mL). Next, the organic layer was dried over MgSO₄ and *concentrated in vacuo.* The crude product was purified by column chromatography (EtOAc/*n*-heptane, 1:4) to obtain compound 10c as yellow oil (444 mg, 87%). *R*_{F} = 0.55 (EtOAc/*n*-heptane, 1:2). ¹H-NMR (300 MHz, CD₃OD) δ: 7.87 (d, *J=* 8.3 Hz, 1H), 7.71 (m, 1H), 7.50-7.48 (m, 1H), 7.46-7.41 (m, 2H), 7.38-7.32 (m, 1H), 7.08 (td, *J =* 8.7, 1.2 Hz, 1H), 4.85 (s, 2H), 1.54 (s, 9H). ¹³C-NMR (75 MHz, CD₃OD) δ: 154.9, 146.8, 140.8, 134.4, 132.9, 131.3, 131.0, 130.7, 124.4, 124.1, 121.7, 121.0, 115.0, 92.9, 91.9, 81.8, 63.2, 28.7 (3C). FT-IR vₘₐₓ film (cm⁻¹): 3395, 2976, 2928, 2366, 1735, 1519, 1498, 1455, 1394, 1243, 1161, 1044, 746. HRMS (ESI+) *m*/*z* calcd for C₂₀H₂₀BrNNaO₃ [M+Na]⁺ 424.0524, found 424.0513.

### Example 17: tert-Butyl (2-((2-(hydroxymethyl)-4-nitrophenyl)ethynyl)phenyl)-carbamate (10d)

Compound **9d** (100 mg, 0.37 mmol) was dissolved in THF (370 µL) and Boc₂O (81 mg, 0.37 mmol) was added. The reaction was stirred in a sealed tube at 70 °C overnight. The reaction mixture was diluted with CH₂Cl₂ (10 mL) and the organic layer was washed with H₂O (3 × 10 mL) and brine (10 mL) and subsequently dried over MgSO₄. The solvents were *removed in vacuo* and the crude product was purified by gradient column chromatography (EtOAc/*n*-heptane, 1:6 to 1:4). Compound **10d** was obtained as red solid (70 mg, 51%). In addition **9d** was reobtained (40 mg, 40%). ¹H-NMR (400 MHz, CDCl₃) δ: 8.36 (d, *J=* 1.6 Hz, 1H), 8.20 (dd, *J* = 8.5, 2.3 Hz, 1H), 8.15 (br s, 1H), 7.80 (s, 1H), 7.71 (d, *J =* 8.5 Hz, 1H), 7.49 (d, *J* = 7.7 Hz, 1H), 7.40 (t, *J =* 7.9 Hz, 1H), 7.04 (t, *J =* 7.6 Hz, 1H), 5.00 (d, *J =* 4.6 Hz, 2H), 2.59 (br s, 1H), 1.58 (s, 9H). ¹³C-NMR (75 MHz, CDCl₃) δ: 152.5, 147.1, 142.9, 140.5, 132.7, 131.8, 130.9, 123.1, 122.9, 122.4, 118.3, 114.6, 110.4, 94.6, 92.3, 81.6, 63.5, 28.3 (3C). HRMS (ESI+) *m*/*z* calcd for C₂₀H₂₀N₂NaO₅ [M+Na]⁺ 391.1270, found 391.1265.

### Example 18: (Z)-tert-Butyl (2-(5-chloro-2-(hydroxymethyl)styryl)phenyl)carbamate (11a)

Compound **10a** (8.24 g, 23.1 mmol) was dissolved in methanol (100 mL). After addition of quinoline (273 µl, 2.31 mmol) and 10% Pd/BaSO₄ (492 mg, 0.231 mmol), the reaction was stirred under H₂-atmosphere for 2 hours. The reaction mixture was then filtered over celite and diluted with CH₂Cl₂ (150 mL). The organic layer was washed with 2 M aqueous HCl (2 × 100 mL), H₂O (100 mL), and brine (100 mL). The organic layer was dried over MgSO₄ and the volatiles were removed under reduced pressure to obtain compound **11a** (7.91 g, 95%). ¹H-NMR (400 MHz, CDCl₃) δ: 7.26-7.18 (m, 2H), 7.14 (dd, *J =* 8.2, 1.9 Hz, 1H), 7.11 (s, 1H), 6.99 (t, *J =* 7.4 Hz, 1H), 6.92 (s, 1H), 6.90 (d, *J =* 12.0 Hz, 1H), 6.69 (d, *J =* 12.0 Hz, 1H), 6.62 (br s, 1H), 4.67 (d, *J =* 6.2 Hz, 2H), 1.43 (s, 9H). ¹³C-NMR (75 MHz, CDCl₃) δ: 150.3, 137.2, 137.0, 134.9, 133.3, 130.0, 129.8, 129.5, 129.3, 128.8, 128.4, 127.8 (2C), 125.8, 123.1, 120.4, 81.0, 63.2, 28.2. HRMS (ESI+) *m*/*z* calcd for C₂₀H₂₃ClNO₃ [M+H]⁺ 360.1367, found 360.1387.

### Example 19: (Z)-tert-Butyl (2-(4-bromo-5-chloro-2-(hydroxymethyl)styryl)phenyl)-carbamate (11b)

Compound **10b** (470 mg, 1.1 mmol) was dissolved in methanol (20 mL) and 10% Pd/BaSO₄ (15 mg, 14 µmol) and quinoline (13 µl, 0.11 mmol) were added. The reaction was stirred under H₂-atmosphere for two hours. Additional 10% Pd/BaSO₄ (15 mg, 14 µmol) was added, and after 1 hour again 10% Pd/BaSO₄ (15 mg, 14 µmol) was added. After 1 additional hour the reaction was completed and filtered over celite. The celite was washed with CH₂Cl₂ (50 mL), and the thus obtained organic layer was washed with 2M aqueous HCl (2 × 50 mL), H₂O (50 mL), and brine (50 mL) and subsequently dried over MgSO₄. The solvents were removed under reduced pressure to obtain **11b** as a single product (470 mg, 100%). ¹H-NMR (400 MHz, CDCl₃) δ: 8.12 (d, *J =* 8.5 Hz, 1H), 7.74 (br s, 1H), 7.72 (m, 1H), 7.63 (s, 1H), 7.44 (ddd, *J =* 7.7, 1.6, 0.5 Hz, 1H), 7.36 (dddd, *J =* 7.5, 1.6, 0.5 Hz, 8.5 Hz, 1H), 7.01 (dt, *J =* 7.5, 1.1 Hz, 1H), 4.85 (d, *J =* 4.8 Hz, 2H), 2.45 (br s, 1H), 1.57 (s, 9H). ¹³C-NMR (75 MHz, CDCl₃) δ: 152.8, 138.7, 135.9, 134.8, 133.3, 130.3, 129.2, 129.0, 128.6, 128.3 (2C), 125.7, 123.4, 121.4, 120.8, 81.2, 62.5, 28.2 (3C). HRMS (ESI+) *m*/*z* calcd for C₂₀H₂₂BrClNO₃ [M+H]⁺ 438.0472, found 438.0495.

### Example 20: (Z)-tert-butyl 2-(4-bromo-2-(hydroxymethyl)styryl)phenylcarbamate (11c)

Compound **10c** (720 mg, 1.8 mmol) was dissolved in methanol (12 mL) and 10% Pd/BaSO₄ (35 mg, 33 µmol) and quinoline (21 µL, 0.18 mmol) were added. The reaction mixture was stirred under H₂-atmosphere for 1.5 hour. Next, the mixture was filtered over celite and the solvents were removed under reduced pressure. The crude product was purified by column chromatography (EtOAc/*n*-heptane, 1:9) to obtain compound **11c** as orange oil (650 mg, 90%). *R*_{F} = 0.40 (EtOAc/*n*-heptane, 1:2). ¹H-NMR (400 MHz, CDCl₃) δ: 7.48 (s, 1H), 7.21 (t, *J* = 7.5 Hz, 1H), 7.14-7.12 (m, 2H), 6.99 (t, *J =* 7.3 Hz, 1H), 6.88 (d, *J =* 12.1 Hz, 1H), 6.81 (d, *J =* 7.6 Hz, 1H), 6.64 (d, *J* = 11.9 Hz, 1H), 6.62 (s, 1H), 4.70 (d, *J* = 6.4 Hz, 2H), 1.43 (s, 9H). ¹³C-NMR (75 MHz, CDCl₃) δ: 152.6, 140.5, 134.8, 134.3, 131.3, 130.7, 130.5, 130.2, 129.4, 128.3, 128.0, 127.3, 123.0, 121.7, 120.2, 81.2, 63.3, 28.2 (3C). FT-IR vₘₐₓ film (cm⁻¹): 3421, 2976, 2933, 2362, 2327, 1705, 1576, 1519, 1472, 1446, 1398, 1364, 1308, 1230, 1156, 1053, 1022, 767, 763. HRMS (ESI+) *m*/*z* calcd for C₂₀H₂₃BrNO₃ [M+H]⁺ 404.0861, found 404.0865.

### Example 21: (Z)-tert-Butyl(2-(2-(hydroxymethyl)-4-nitrostyryl)phenyl)carbamate (11d)

Compound **10d** (70 mg, 0.19 mmol) was dissolved in methanol (10 mL) and quinoline (11 µL, 95 µmol) and 10% Pd/BaSO₄ (2.66 mg, 2 µmol) were added. The reaction was stirred under H₂- atmosphere for 3 hours after which the mixture was filtered over celite. The celite was washed with CH₂Cl₂ (20 mL) and the organic layer was washed with H₂O (2 × 20 mL) and brine (20 mL) and subsequently dried over MgSO₄. The solvents were removed under reduced pressure and the crude product was purified by column chromatography (EtOAc/*n*-heptane, 1:4) to obtain **11d** as a red solid (55 mg, 78%). ¹H-NMR (300 MHz, CDCl₃) δ: 8.24 (d, *J=* 2.4 Hz, 1H), 7.84 (dd, *J=* 8.5, 2.4 Hz, 1H), 7.73 (br s, 1H), 7.26-7.18 (m, 1H), 7.10 (d, *J* = 8.5 Hz, 1H)), 7.06 (s, 1H), 6.98 (d, *J* = 7.4 Hz, 1H), 6.93 (d, *J* = 12.0 Hz, 1H), 6.81 (d, *J* = 12.0 Hz, 1H), 6.56 (s, 1H), 4.79 (s, 2H), 1.40 (s, 9H). ¹³C-NMR (75 MHz, CDCl₃) δ: 152.6, 147.0, 142.2, 140.3, 135.0, 129.9, 129.7, 129.3 (2C), 128.8, 125.7, 123.4, 123.1, 122.4, 120.8, 81.3, 62.9, 28.13 (3C). HRMS (ESI+) *m*/*z* calcd for C₂₀H₂N₂NaO₅ [M+Na]⁺ 393.14264, found 393.14315.

### Example 22: (Z)-tert-butyl (2-(5-chloro-2-formylstyryl)phenyl)carbamate (12a)

Compound **11a** (7.91 g, 22 mmol) was dissolved in dry CH₂Cl₂ (150 mL) under Ar-atmosphere in a flame-dried flask. Dess-Martin periodinane (11.2 g, 26.4 mmol) and NaHCO₃ (5.54 g, 66 mmol) were added and the mixture was stirred for 45 minutes. The reaction was quenched by the addition of saturated aqueous NaHSO₃ (100 mL). The layers were separated and the H₂O-layer was extracted with CH₂Cl₂ (100 mL). The organic layers were combined and washed with saturated aqueous NaHSO₃ (200 mL), H₂O (2 × 200 mL) and brine (200 mL) and then dried over MgSO₄. The organic solvents were evaporated and the crude product was purified by column chromatography (EtOAc/*n*-heptane, 1:9). Compound **12a** was obtained as a yellow solid (7.36 g, 95%). ¹H-NMR (300 MHz, CDCl₃) δ: 10.13 (s, 1H), 7.87 (d, *J=* 8.2 Hz, 1H), 7.74 (d, *J* = 8.3 Hz, 1H), 7.33 (dd, *J* = 8.1, 1.9 Hz, 1H), 7.25-7.18 (m, 1H), 7.16 (d, *J =* 12.0 Hz, 1H), 7.09 (d, *J =* 2.0 Hz, 1H), 7.00-6.87 (m, 2H), 6.84 (d, *J =* 11.9 Hz, 1H), 6.44 (br s, 1H), 1.46 (s, 9H). ¹³C-NMR (75 MHz, CDCl₃) δ: 190.6, 152.4, 140.4, 140.0, 135.6, 132.3, 131.7, 130.3, 129.7, 129.5, 129.0, 128.9, 128.3, 125.1, 123.1, 120.3, 80.5, 28.3 (3C). HRMS (ESI+) *m*/*z* calcd for C₂₀H₂₀ClNNaO₃ [M+Na]⁺ 380.1029, found 380.1032.

### Example 23: (Z)-tert-Butyl (2-(4-bromo-5-chloro-2-formylstyryl)phenyl)carbamate (12b)

Compound **11b** (1.17 g, 2.67 mmol) was dissolved in dry CH₂Cl₂ (40 mL) under Ar-atmosphere in a flame-dried flask. NaHCO₃ (670 mg, 8.0 mmol) and Dess-Martin periodinane (1.47 g, 3.47 mmol) were added and the reaction was stirred for 1 hour. Hereupon, the reaction was quenched with saturated aqueous NaHSO₃ and diluted with CH₂Cl₂ (20 mL). The organic layer was washed with H₂O (3 × 50 mL) and brine (50 mL) and subsequently dried over MgSO₄. The solvents were removed under reduced pressure and the crude product was purified by column chromatography (EtOAc/*n*-heptane, 1:9). Compound **12b** was obtained as a yellow solid (1.02 g, 89%). ¹H-NMR (400 MHz, CDCl₃) δ: 10.07 (s, 1H), 8.02 (s, 1H), 7.85 (d, *J* = 8.2 Hz, 1H), 7.26-7.22 (m, 1H), 7.21 (s, 1H), 7.07 (d, *J =* 11.9 Hz, 1H), 6.98-6.90 (m, 2H), 6.87 (d, *J* = 11.9 Hz, 1H), 6.39 (s, 1H), 1.47 (d, *J* = 1.0 Hz, 9H). ¹³C-NMR (75 MHz, CDCl₃) δ: 189.3, 152.4, 140.1, 138.8, 135.6 (2C), 132.7, 131.9, 130.5, 129.5, 129.1, 127.6, 125.1, 123.4, 122.2, 120.7, 80.7, 28.3 (3C). HRMS (ESI+) m/z calcd for C₂₀H₁₉BrClNNaO₃ [M+Na]⁺ 458.0135, found 458.0123.

### Example 24: (Z)-tert-Butyl 2-(4-bromo-2-formylstyryl)phenylcarbamate (12c)

Compound **11c** (651 mg, 1.62 mmol) was dissolved in dry CH₂Cl₂ (15 mL) and placed under an Ar-atmosphere in a flame-dried flask. Subsequently, Dess-Martin periodinane (888 mg, 2.09 mmol) and NaHCO₃ (406 mg, 4.83 mmol) were added and the mixture was stirred for 40 minutes. The reaction was quenched with saturated aqueous Na₂SO₃ (15 mL). The mixture was diluted with CH₂Cl₂ (25 mL), washed with saturated aqueous NaHSO₃ (15 mL), H₂O (15 mL) and brine (15 mL). Next, the organic layer was dried over MgSO₄ and *concentrated in vacuo.* The crude product was purified by column chromatography (EtOAc/*n*-heptane, 1:9) to obtain compound **12c** as a yellow oil which solidified upon storage at -20 °C (560 mg, 86%). *R*_{F} = 0.40 (EtOAc/*n*-heptane, 1:4). ¹H-NMR (400 MHz, CDCl₃) δ: 10.16 (s, 1H), 7.93 (d, *J=* 2.2 Hz, 1H), 7.87 (d, *J =* 7.7 Hz, 1H), 7.43 (dd, *J =* 8.3, 2.2 Hz, 1H), 7.23-7.19 (m, 1H), 7.17 *(d, J =* 12.0 Hz, 1H), 6.99-6.96 (m, 2H), 6.92 (d, *J =* 7.3 Hz, 1H), 6.82 (d, *J* = 11.9 Hz, 1H), 6.42, (br s, 1H), 1.45 (s, 9H). ¹³C-NMR (75 MHz, CDCl₃) δ: 190.4, 152.4, 137.5, 136.4, 135.5, 134.7, 133.8, 131.9, 129.6, 129.3, 129.2, 128.7, 125.5, 123.1, 122.0, 120.3, 80.6, 28.2 (3C). FT-IR vₘₐₓ film (cm⁻¹): 2982, 1729, 1695, 1584, 1515, 1445, 1238, 1369, 1148, 1051, 1016, 780, 746. HRMS (ESI+) *m*/*z* calcd for C₂₀H₂₀BrNNaO₃ [M+Na]⁺ 424.0524, found 424.0516.

### Example 25: (Z)-tert-Butyl (2-(2-formyl-4-nitrostyryl)phenyl)carbamate (12d)

Compound **11d** (170 mg, 0.46 mmol) was dissolved in dry CH₂Cl₂ (5 mL) under Ar-atmosphere in a flame-dried flask. Dess-Martin periodinane (234 mg, 0.55 mmol) and NaHCO₃ (116 mg, 1.38 mmol) were added. The reaction was stirred for 30 minutes whereupon saturated aqueous NaHSO₃ (10 mL) was added. The mixture was diluted with CH₂Cl₂ (15 mL) and the organic layer was washed with saturated aqueous NaHSO₃ (30 mL), water (2 × 30 mL) and brine (30 mL) before drying over MgSO₄. The solvents were *removed in vacuo* and the crude product was purified by gradient column chromatography (EtOAc/*n*-heptane, 1:19 to 1:6) to obtain 12d as a red solid (145 mg, 86%). ¹H-NMR (400 MHz, CDCl₃) δ: 10.27 (s, 1H), 8.66 (d, *J=* 2.4 Hz, 1H), 8.14 (dd, *J =* 8.5, 2.5 Hz, 1H), 7.82 (d, *J =* 8.3 Hz, 1H), 7.32 (d, *J =* 8.6 Hz, 1H), 7.25 *(d, J=* 11.8 Hz, 1H), 7.26-7.21 (m, 1H), 6.97 (d, *J* = 11.9 Hz, 1H), 6.93-6.88 (m, 2H), 6.44-6.35 (br s, 1H), 1.45 (s, 9H). ¹³C-NMR (75 MHz, CDCl₃) δ: 189.7, 152.4, 144.8, 135.7, 134.1, 133.0, 131.9, 131.6, 129.6, 129.3, 128.4, 127.4, 126.0, 125.4, 123.5, 121.0, 80.8, 28.3 (3C). HRMS (ESI+) *m*/*z* calcd for C₂₀H₂₀N₂NaO₅ [M+Na]⁺ 391.1270, found 391.1259.

### Example 26: (Z)-9-Chloro-5,6-dihydrodibenzo[b,f]azocine (13a)

Compound **12a** (7.34 g, 20.6 mmol) was dissolved in 2M HCl in EtOAc (100 ml, 200.00 mmol) and the reaction was stirred for 1 hour. Then, NaBH₄ (3.11 g, 82.4 mmol) in H₂O (10 mL) was added and the reaction was stirred overnight. As reduction of the imine was not completed yet, another portion of NaBH₄ (2.25 g, 60 mmol) was added and the reaction was stirred for 1.5 hour. Hereupon, the reaction was quenched by the addition of H₂O (50 mL) and the product was extracted with EtOAc (100 mL). The organic layer was washed with H₂O (3 × 100 mL) and brine (100 mL) and subsequently dried over MgSO₄. The solvents were removed under reduced pressure to obtain **13a** as a yellow solid (4.7 g, 95%). ¹H-NMR (300 MHz, CDCl₃) δ: 7.18-7.09 (m, 3H), 7.06-6.84 (m, 2H), 6.69-6.60 (m, 1H), 6.58 (d, *J =* 13.2 Hz, 1H), 6.46 (dd, *J* = 8.1, 0.6 Hz, 1H), 6.27 (d, *J* = 13.1 Hz, 1H), 4.55 (s, 2H), 4.22 (br s, 1H). ¹³C-NMR (75 MHz, CDCl₃) δ: 146.8, 141.1, 136.6, 134.7, 134.0, 133.3, 130.3, 129.7, 128.3, 127.3, 126.1, 121.3, 118.0, 117.9, 48.8. HRMS (ESI+) *m*/*z* calcd for C₁₅H₁₃ClN [M+H]⁺ 242.0737, found 242.0726.

### Example 27: (Z)-8-Bromo-9-chloro-5,6-dihydrodibenzo[b,f]azocine (13b)

Compound **12b** (920 mg, 2.1 mmol) was dissolved in 2M HCl in EtOAc (40 mL, 80 mmol). After 30 minutes, NaBH₄ (240 mg, 6.3 mmol) in H₂O (2 mL) was added. After two hours another portion of NaBH₄ (240 mg, 6.3 mmol) in H₂O (2 mL) was added, followed by another portion of NaBH₄ (80 mg, 2.1 mmol) in H₂O (1 mL) after 1 hour. After an extra 30 minutes, the reaction was quenched by the addition of H₂O (40 mL). The layers were separated, and the H₂O-layer was extracted with EtOAc (50 mL). The organic layers were combined and washed with H₂O (2 × 100 mL), and brine (100 mL), and subsequently dried over MgSO₄. The volatiles were removed under reduced pressure and the crude product was purified by column chromatography (EtOAc/*n*-heptane, 1:6). Compound 13b was obtained as yellow solid (340 mg, 50%). ¹H-NMR (300 MHz, CDCl₃) δ: 7.44 (s, 1H), 7.24 (s, 1H), 6.99-6.87 (m, 2H), 6.63 (dt, *J =* 7.7, 1.3 Hz, 1H), 6.59 (d, *J =* 13.0 Hz, 1H), 6.47 (dd, *J =* 8.0, 1.2 Hz, 1H), 6.20 (d, *J=* 13.1 Hz, 1H), 4.52 (s), 4.24 (s). ¹³C-NMR (75 MHz, CDCl₃) δ: 146.6, 140.1, 138.4,134.7, 134.4, 134.0, 133.4, 131.5, 128.5, 125.1, 121.3, 120.1, 118.1, 118.0, 48.6. HRMS (ESI+) *m*/*z* calcd for C₁₅H₁₂BrClN [M+H]⁺ 319.9842, found 319.9825.

### Example 28: (Z)-8-Bromo-5,6-dihydrodibenzo[b,f]azocine (13c)

Compound **12c** (560 mg, 1.4 mmol) was dissolved in 2M HCl in EtOAc (20 mL, 40 mmol) and stirred for 1.5 hour. Next, NaBH₄ (196 mg, 5.2 mmol) and a few drops of water were added. The reaction was stirred overnight whereupon an additional portion of NaBH₄ (196 mg, 5.2 mmol) was added and, after an additional 90 minutes, the reaction was quenched with H₂O (15 mL). The H₂O-layer was extracted with EtOAc (2 × 15 mL). The organic layers were combined and washed with 2M aqueous NaOH (2 × 20 mL), H₂O (2 × 20 mL) and brine (20 mL). Next, the organic layer was dried over MgSO₄ and *concentrated in vacuo* to obtain compound 13c as a yellow solid (360 mg, 91%). *R*_{F} = 0.55 (EtOAc/*n*-heptane, 1:2). ¹H-NMR (400 MHz, CDCl₃) δ: 7.36 (dd, *J =* 8.7, 2.1 Hz, 1H), 7.34 (d, *J =* 2.1 Hz, 1H), 7.02 (d, *J =* 8.1 Hz, 1H), 6.96 (dd, *J* = 7.7, 1.7 Hz, 1H), 6.90 (ddd, *J* = 7.2, 6.5, 1.6 Hz, 1H), 6.62 (ddd, *J* = 8.4, 7.2, 1.2 Hz, 1H), 6.55 (d, *J =* 13.1 Hz, 1H), 6.47 (dd, *J =* 8.1, 1.2 Hz, 1H), 6.26 (d, *J* = 13.1 Hz, 1H), 4.54 (s, 2H). ¹³C-NMR (75 MHz, CDCl₃) δ: 146.3, 139.8, 137.7, 134.3, 132.9, 131.3 (2C), 130.2, 127.7, 126.9, 125.8, 121.1, 119.8, 117.5, 48.7. FT-IR vₘₐₓ film (cm⁻¹): 3391, 3002, 2924, 2850, 2362, 2098, 1593, 1485, 1325, 1269, 901, 828, 776, 750. HRMS (ESI+) m/z calcd for C₁₅H_{113b}rN [M+H]⁺ 286.02314 found 286.0219.

### Example 29: (Z)-8-Nitro-5,6-dihydrodibenzo[b,f]azocine (13d)

Compound **12d** (200 mg, 0.54 mmol) was dissolved in HCl in EtOAc (10 mL, 20 mmol). After 30 minutes NaBH₄ (470 mg, 12.4 mmol) in water (1 mL) was added and after stirring overnight the reaction was quenched with H₂O (10 mL). The H₂O-layer was extracted with EtOAc (20 mL), and the combined organic layers were washed with H₂O (3 × 20 mL), brine (20 mL) and subsequently dried over MgSO₄. The solvents were *removed in vacuo* to obtain 13d as a red solid (140 mg, 100%) ¹H-NMR (400 MHz, CDCl₃) δ: 8.11 (dd, *J =* 8.5, 2.4 Hz, 1H), 8.05 (d, *J =* 2.3 Hz, 1H), 7.29 (d, *J =* 8.6 Hz, 1H), 7.01 (d, *J =* 7.7 Hz, 1H), 6.97-6.91 (m, 1H), 6.71-6.62 (m, 2H), 6.51 (d, *J =* 8.1 Hz, 1H), 6.36 (d, *J =* 13.4 Hz, 1H), 4.69 (s, 2H). ¹³C-NMR (75 MHz, CDCl₃) δ: 147.3, 146.7, 145.9, 139.5, 135.9, 135.4, 131.3, 128.8, 125.0, 124.1, 122.8, 121.2, 118.4, 118.0, 49.6. HRMS (ESI+) *m*/*z* calcd for C₁₅H₁₃N₂O₂ [M+H]⁺ 253.0977 found 253.0965.

### Example 30: (Z)-Methyl 5- (9-chlorodibenzo [b,f] azocin-5(6H)-yl)-5-oxopentanoate (14a)

Compound **13a** (3 g, 12.4 mmol) was dissolved in dry CH₂Cl₂ (100 mL) and NEt₃ (3.46 mL, 24.8 mmol) was added. After cooling the mixture to 0 °C, methyl 5-chloro-5-oxopentanoate (2.13 mL, 15 mmol) was added and the reaction was stirred overnight. Hereupon, the reaction was quenched with H₂O (100 mL) and the layers were separated. The organic layer was washed with 2M aqueous NaOH (2 × 70 mL), water (2 × 70 mL) and brine (70 mL) and subsequently dried over MgSO₄. The solvents were removed under reduced pressure and the crude product was purified by column chromatography (EtOAc/*n*-heptane, 1:2) to obtain **14a** as a yellow solid (2.11 g, 46%). ¹H-NMR (400 MHz, CDCl₃) δ: 7.31-7.27 (m, 3H), 7.23 *(d, J=* 8.8 Hz, 1H), 7.19-7.10 (m, 3H), 6.69 (d, *J* = 13.1 Hz, 1H), 6.62 (d, *J* = 13.1 Hz, 1H), 5.43 (d, *J =* 14.9 Hz, 1H), 4.17 *(d, J=* 14.9 Hz, 1H), 3.59 (s, 3H), 2.25-2.03 (m, 3H), 1.94-1.76 (m, 3H). ¹³C-NMR (75 MHz, CDCl₃) δ: 173.5, 171.8, 140.8, 137.6, 135.8, 133.3, 132.7, 131.7, 131.5, 131.4, 131.1, 128.7, 128.5, 128.1, 128.1, 127.3, 53.9, 51.4, 33.5, 33.0, 20.4. HRMS (ESI+) *m*/*z* calcd for C₂₁H₂₁ClNO₃ [M+H]⁺ 370.1210, found 370.1203.

### Example 31: (Z)-Methyl 5-(8-bromo-9-chlorodibenzo[b,f]azocin-5(6H)-yl)-5-oxopentanoate (14b)

Compound **13b** (200 mg, 0.62 mmol) was dissolved in dry CH₂Cl₂ (15 mL) and the solution was cooled to 0 °C. Subsequently, NEt₃ (174 µL, 1.25 mmol) and methyl 5-chloro-5-oxopentanoate (133 µl, 0.94 mmol) were added. The reaction was stirred overnight and then quenched with H₂O (10 mL). The layers were separated, and the H₂O-layer was extracted with CH₂Cl₂ (20 mL). The organic layers were combined and washed with H₂O (2 × 30 mL), and brine (30 mL) and dried over MgSO₄. The solvents were *removed in vacuo* and the crude product was purified by column chromatography (EtOAc/*n*-heptane, 1:2). Compound **14b** was obtained as yellow solid (230 mg, 82%). ¹H-NMR (400 MHz, CDCl₃) δ: 7.53 (s, 1H), 7.34-7.29 (m, 3H), 7.22 (s, 1H), 7.18 (dd, *J* = 6.4, 2.6 Hz, 1H), 6.64 (d, *J* = 13.3 Hz, 1H), 6.60 (d, *J =* 13.3 Hz, 1H), 5.45 (d, *J* = 15.2 Hz, 1H), 4.12 (d, *J* = 15.2 Hz, 1H), 3.59 (s, 3H), 2.62-2.29 (m, 1H), 2.26-1.96 (m, 2H), 1.93-1.76 (m, 3H). ¹³C-NMR (75 MHz, CDCl₃) δ: 173.4, 171.8, 140.5, 136.2, 135.5, 135.0, 134.9, 133.2, 132.7, 131.4, 130.0, 129.0, 128.7, 128.2, 128.1, 120.8, 53.4, 51.3, 33.3, 32.9, 20.3. HRMS (ESI+) *m*/*z* calcd for C₂₁H₂BrClNO₃ [M+H]⁺ 448.0321, found 448.0315.

### Example 32: (Z)-Methyl 5-(8-bromodibenzo[b,f]azocin-5(6H)-yl)-5-oxopentano-ate (14c)

Compound **13c** (360 mg, 1.26 mmol) was dissolved in dry CH₂Cl₂ (8 mL) and NEt₃ (351 µL, 2.52 mmol) was added. The mixture was cooled to 0 °C, whereupon methyl 5-chloro-5-oxopentanoate (221 µL, 1.89 mmol) was added. The reaction was stirred for 90 minutes, after which it was quenched with H₂O (5 mL). The mixture was diluted with CH₂Cl₂ (10 mL) and washed with 2M aqueous NaOH (2 × 10 mL), 2M aqueous HCl (2 × 10 mL), H₂O (2 × 10 mL) and brine (10 mL). Next, the organic layer was dried over MgSO₄ and *concentrated in vacuo.* The crude product was purified by column chromatography (EtOAc/*n*-heptane, 1:2) to obtain compound 14c as a yellow solid (466 mg, 90%). *R*_{F} = 0.30 (EtOAc/*n*-heptane, 1:2). ¹H-NMR (400 MHz, CDCl₃) δ: 7.42 (d, *J=* 2.1 Hz, 1H), 7.31-7.27 (m, 4H), 7.19-7.15 (m, 1H), 7.00 (d, *J* = 8.3 Hz, 1H), 6.69 (d, *J* = 13.1 Hz, 1H), 6.59 (d, *J* = 13.1 Hz, 1H), 5.49 (d, *J =* 15.2 Hz, 1H), 4.16 (d, *J* = 15.2 Hz, 1H), 3.59 (s, 3H), 2.22-2.17 (m, 2H), 2.13-2.04 (m, 2H), 1.85-1.79 (m, 2H). ¹³C-NMR (75 MHz, CDCl₃) δ: 173.5, 171.8, 140.7, 136.9, 136.1, 134.5, 133.7, 132.9, 131.4, 131.1, 130.1, 128.8, 128.1 (2C), 127.6, 121.2, 54.0, 51.4, 33.3, 33.1, 20.4. FT-IR vₘₐₓ film (cm⁻¹): 3473, 2947, 2868, 2150, 1874, 1731, 1653, 1498, 1437, 1403, 1195, 1169, 1018, 832, 776. HRMS (ESI+) *m*/*z* calcd for C₂₁H₂₁BrNO₃ [M+H]⁺ 414.0705, found 414.0699.

### Example 33: (Z)-Methyl 5-(8-nitrodibenzo[b,f]azocin-5(6H)-yl)-5-oxopentanoate (14d)

Compound **13d** (45 mg, 0.18 mmol) was dissolved in dry CH₂Cl₂ (5 ml) and the solution was cooled to 0 °C. Subsequently, NEt₃ (50 µL, 0.36 mmol) and methyl 5-chloro-5-oxopentanoate (37 µl, 0.27 mmol) were added. The reaction was stirred overnight and quenched by the addition of 0.1 M aqueous NaOH (5 mL). The reaction was diluted with CH₂Cl₂ (10 mL) and the organic layer was washed with 2M aqueous NaOH (2 × 20 mL), H₂O (20 mL) and brine (20 mL), and subsequently dried over MgSO₄. The solvents were removed under reduced pressure and the crude product was purified by gradient column chromatography (EtOAc/*n*-heptane, 1:6 to 2:3). Compound **14d** was obtained as a red solid (25 mg, 37%). ¹H-NMR (300 MHz, CDCl₃) δ: 8.20 (d, *J*= 2.4 Hz, 1H), 8.02 (dd, *J* = 8.5, 2.4 Hz, 1H), 7.32 (dt, *J* = 11.5, 4.1 Hz, 5H), 7.24-7.17 (m, 1H), 6.81 (d, *J* = 13.4 Hz, 1H), 6.73 (d, *J* = 13.3 Hz, 1H), 5.55 (d, *J* = 15.1 Hz, 1H), 4.23 (d, *J* = 15.0 Hz, 1H), 3.58 (s, 3H), 2.25-2.08 (m, 2H), 2.06-1.91 (m, 2H), 1.86-1.66 (m, 2H). HRMS (ESI+) *m*/*z* calcd for C₂₁H₂₁N₂O₅ [M+H]⁺ 381.1451, found 381.1446.

### Example 34: Methyl 5-(11,12-dibromo-9-chloro-11,12-dibenzo[b,f]azocin-5(6H)-yl)-5-oxopentanoate (15a)

Compound **14a** (1 g, 2.7 mmol) was dissolved in CH₂Cl₂ (50 mL) and the solution was cooled to 0 °C. A solution of Br₂ (154 µl, 3 mmol) in CH₂Cl₂ (5 mL) was added dropwise and after 2 hours at 0 °C additional Br₂ (20 µL, 0.39 mmol) in CH₂Cl₂ (1 mL) was added. After 1 hour the reaction was quenched by addition of saturated aqueous NaHSO₃ (50 mL), and layers were separated. The organic layer was washed with saturated aqueous NaHSO₃-solution (50 mL), water (2 × 50 mL) and brine (50 mL) and subsequently dried over MgSO₄. The volatiles were removed under reduced pressure and the crude product was purified by gradient column chromatography (EtOAc/*n*-heptane, 1:4 to 1:2) to obtain compound **15a** (1.02 g, 71%) as mixture of two diastereoisomers (**X** : **Y**, 1:0.8). The isomers could be separated however due to slow isomerization of **X** to **Y**, no full analysis of **15aY** was performed. Analytical data for **15aX:** *R*_{F} = 0.30 (EtOAc/*n*-heptane, 1:2). ¹H-NMR (300 MHz, CDCl₃) δ: 7.72 (d, *J* = 2.1 Hz, 1H), 7.28-7.15 (m, 2H), 7.12-6.94 (m, 3H), 6.83 (d, *J* = 8.3 Hz, 1H), 5.85 (d, *J* = 9.9 Hz, 1H), 5.77 (d, *J* = 14.8 Hz, 1H), 5.12 (d, *J* = 10.0 Hz, 1H), 4.14 (d, *J* = 14.8 Hz, 1H), 3.61 (s, 3H), 2.97 - 2.27 (m, 3H), 2.27 - 2.13 (m, 1H), 2.08 - 1.92 (m, 2H). ¹³C-NMR (75 MHz, CDCl₃) δ: 173.6, 172.7, 138.9, 137.8, 137.0, 134.7, 131.5, 130.9 (2C), 130.7, 130.4, 129.6, 129.0, 128.9, 59.5, 54.5, 51.8, 51.5, 34.8, 33.3, 20.3. HRMS (ESI+) *m*/*z* calcd for C₂₁H₂₁Br₂ClNO₃ [M+H]⁺ 527.9577, found 527.9564. **15aY:** *R*_{F} = 0.35 (EtOAc/*n*-heptane, 1:2). ¹H-NMR (400 MHz, CDCl) δ 7.65 (dd, *J* = 7.8, 1.3 Hz, 1H), 7.25-6.98 (m, 4H), 6.88-6.78 (m, 2H), 5.71 (d, *J* = 9.6 Hz, 1H), 5.14 (d, *J* = 9.6 Hz, 1H), 5.13 (d, *J* = 14.5 Hz, 1H), 4.95 (d, *J* = 14.0 Hz, 1H), 3.63 (s, 3H), 2.48-2.31 (m, 3H), 2.26-2.05 (m, 1H), 2.04-1.80 (m, 2H).

### Example 35: Methyl 5-oxo-5-(8,11,12-tribromo-9-chloro-11,12-dibenzo[b,f]azocin-5(6H)-yl)pentanoate (15b)

Compound **14b** (100 mg, 0.22 mmol) was dissolved in CH₂Cl₂ (10 mL) and the solution was cooled to 0 °C. A solution of Br₂ (11 µl, 0.22 mmol) in CH₂Cl₂ (1 mL) was added dropwise and the reaction was stirred at 0 °C. After 2 hours, the reaction was quenched with saturated aqueous NaHSO₃ (10 mL). The layers were separated and the H₂O-layer was extracted with CH₂Cl₂ (10 mL). The combined organic layers were washed with saturated aqueous NaHSO₃ (20 mL), water (2 × 20 mL) and brine (20 mL) and dried over MgSO₄. The solvents were removed under reduced pressure to obtain **15b** as white solid (130 mg, 96%). **15b** was obtained as a mixture of two diastereoisomers **(15bX** : **15bY,** 0.32:1). For the ¹H-NMR signals from **15bX** are designated with *, signals from **15bY** with °. In the ¹³C-NMR data, only peaks are given from major isomer **15bX.** ¹H-NMR (400 MHz, CDCl₃) δ 7.82* (s, 0.3H), 7.65° (d, *J* = 7.7 Hz, 1H), 7.35° (s, 1H), 7.32-7.08*^{,}° (m, 3.4H), 7.02° (s, 1H), 7.00* (s, 0.3H), 6.90° (d, *J* = 7.8 Hz, 1H), 5.80* (d, *J* = 9.8 Hz, 0.3H), 5.79* (d, *J* = 15.1 Hz, 0.3H), 5.69° (d, *J* = 9.6 Hz, 1H), 5.12*^{,}° (d, *J* = 9.5 Hz, 1.3H), 5.08° (d, *J* = 14.6 Hz, 1H), 4.92° (d, *J* = 14.3 Hz, 1H), 4.09* (d, *J* = 15.3 Hz, 0.3H), 3.63° (d, *J* = 0.7 Hz, 3H), 3.62* (s, 1H), 2.60-2.29*^{,}° (m, 4H), 2.21-2.02*^{,}° (m, 1.3H), 2.01-1.83*^{,}° (m, 2.6H). ¹³C-NMR (75 MHz, CDCl₃) δ: 173.6, 172.8, 137.9, 137.7, 136.8, 134.9, 134.2, 133.2, 131.0 (2C), 130.8, 130.5, 130.0, 122.7, 59.1, 53.9, 51.5, 51.3, 34.7, 33.2, 20.3. HRMS (ESI+) *m*/*z* calcd for C₂₁H₂₀Br₃ClNO₃ [M+H]⁺ 605.8682, found 605.8686.

### Example 36: Methyl 5-oxo-5-(8,11,12-tribromo-11,12-dibenzo[b,f]azocin-5(6H)-yl)pentanoate (15c)

Compound **14c** (100 mg, 0.24 mmol) was dissolved in dry CH₂Cl₂ (5 mL). The solution was cooled to 0 °C and Br₂ (13 µL, 0.24 mmol) was added. After stirring for 1.5 hour, additional Br₂ (13 µL, 0.24 mmol) was added and the reaction was stirred for an additional hour. Hereupon, the reaction was quenched with saturated aqueous Na₂SO₃ (5 mL). The mixture was diluted with CH₂Cl₂ (10 mL) and washed with saturated aqueous Na₂SO₃ (2 × 10 mL), H₂O (2 × 10 mL) and brine (10 mL). Next, the organic layer was dried over MgSO₄ and concentrated *in vacuo.* The crude product was purified by gradient column chromatography (EtOAc/*n*-heptane, 1:4 to 1:2) to obtain compound **15c** as a white solid (111 mg, 80%). Compound **15c** was obtained as mixture of diastereoisomers (**15cX : 15cY,** 1:0.47). The major isomer (**15cX**) could be obtained pure, due to slow isomerization of **15cY** to **15cX,** no spectrum of pure **15cY** was obtained. Analytical data for **15cX:** *R*_{F} = 0.25 (EtOAc/*n*-heptane, 1:2). ¹H-NMR (400 MHz, CDCl₃) δ: ¹H-NMR (400 MHz, CDCl₃) δ: 7.61 (d, *J* = 8.4 Hz, 1H), 7.36 - 7.26 (m, 2H), 7.19 (td, *J* = 7.6, 1.4 Hz, 1H), 7.11 - 7.03 (m, 2H), 7.00 (dd, *J* = 7.8, 1.4 Hz, 1H), 5.84 (d, *J* = 10.0 Hz, 1H), 5.82 (d, *J* = 15.1 Hz, 1H), 5.12 (d, *J* = 10.0 Hz, 1H), 4.09 (*d*, *J* = 15.2 Hz, 1H), 3.62 (s, 3H), 2.41-2.27 (m, 3H), 2.25 - 2.14 (m, 1H), 2.09 - 1.94 (m, 2H). ¹³C-NMR (75 MHz, CDCl₃) δ: 173.6, 172.8, 137.8, 136.8, 136.1, 135.0, 132.2, 131.8, 130.8 (2C), 130.5, 130.4, 129.7, 122.6, 59.4, 54.8, 51.9, 51.5, 34.7, 33.2, 20.3. FT-IR νₘₐₓ film (cm⁻¹): 3453, 2953, 2367, 1734, 1660, 1593, 1484, 1441, 1398, 1254, 1203, 1179, 1152, 1015, 875, 844, 801, 769. HRMS (ESI+) *m*/*z* calcd for C₂₁H₂₁Br₃NO₃ [M+H]⁺ 571.9090, found 571.9080. Analytical data for **15cY**: ¹H-NMR (400 MHz, CDCl₃) δ: 7.68-7.61 (m, 1H), 7.38-7.12 (m, 4H), 6.88 (dd, *J* = 7.7, 1.3 Hz, 1H), 6.79 (d, *J* = 8.2 Hz, 1H), 5.72 (d, *J* = 9.4 Hz, 1H), 5.19 (d, *J* = 9.6 Hz, 1H), 5.09 (d, *J* = 14.7 Hz, 2H), 4.96 (d, *J* = 14.1 Hz, 1H), 3.63 (s, 3H), 2.45 - 2.30 (m, 3H), 2.24-2.11 (m, 1H), 2.12-1.83 (m, 2H).

### Example 37: Methyl 5-(11,12-dibromo-8-nitro-11,12-dibenzo[b,f]azocin-5(6H)-yl)-5-oxopentanoate (15d)

Compound **14d** (25 mg, 66 µmol) was dissolved in CH₂Cl₂ (2 mL) and the reaction was cooled to 0 °C. A solution of Br₂ (4.1 µl, 79 µmol) in CH₂Cl₂ (2 mL) was added dropwise. The reaction was stirred at 0 °C for 1 hour, and subsequently quenched with saturated aqueous NaHSO₃ (10 mL) and diluted with CH₂Cl₂ (10 mL). The organic layer was washed with saturated aqueous NaHSO₃ (10 mL), water (2 × 10 mL) and brine (10 mL) and dried over MgSO₄. The solvents were evaporated under reduced pressure to obtain **15d** as a red solid (30 mg, 84%). **15d** was obtained as a mixture of two diastereoisomers **(15dX** : **15dY**, 0.42:1). For the ¹H-NMR signals from **15dX** are designated with *, signals from **15dY** with °. ¹H-NMR (400 MHz, CDCl₃) δ: 8.04* (dd, *J* = 8.6, 2.0 Hz, 0.43H), 7.99-7.90*^{,}° (m, 2.5H), 7.79* (d, *J* = 2.0 Hz, 0.4H), 7.66° (dd, *J* = 7.8, 1.4 Hz, 1H), 7.34-7.14*^{,}° (m, 3.1H), 7.12*^{,}° (*d, J* = 8.4 Hz, 1.2H), 7.09-6.99° (m, 1H), 6.91° (dd, *J* = 7.8, 1.3 Hz, 1H), 5.93* (d, *J* = 11.6 Hz, 0.43H), 5.92* (d, *J* = 13.7 Hz, 0.43H), 5.75° (d, *J* = 9.5 Hz, 1H), 5.28° (d, *J* = 9.6 Hz, 1H), 5.15* (d, *J* = 9.9 Hz, 0.43H), 5.04° (*d*, *J* = 14.3 Hz, 1H), 4.28* (d, *J* = 15.3 Hz, 0.43H), 3.63° (s, 3H), 3.62* (s, 1H), 2.48-2.39*^{,}° (m, 1.45H), 2.39-2.32*^{,}° (m, 3H), 2.17-2.05*^{,}° (m, 1.3H), 2.04-1.89*^{,}° (m, 3.4H). HRMS (ESI+) *m*/*z* calcd for C₂₁H₂₁Br₂N₂O₅ [M+H]⁺ 538.9817, found 538.9822.

### Example 38: Methyl 5-(9-chlorodidehydrodibenzo[b,f]azocin-5(6H)-yl)-5-oxopentanoate (16a)

Compound **15aX** (90 mg, 0.17 mmol) was dissolved in dry THF (3 mL) under Ar-atmosphere in a flame-dried flask. The solution was cooled to -40 °C and a KO*^{t}*Bu-solution in THF (1M, 340 µL, 0.34 mmol) was added dropwise. After stirring at -40 °C for 1.5 hour, additional KO*^{t}*Bu-solution in THF (1M, 50 µL, 0.05 mmol) was added. After 30 minutes, the reaction was quenched with H₂O (5 mL) and diluted with CH₂Cl₂ (10 mL). The layers were separated and the organic layer was washed with H₂O (3 × 15 mL) and brine (15 mL) and subsequently dried over MgSO₄. The solvents were removed under reduced pressure and the crude product was purified by column chromatography (EtOAc/*n*-heptane, 1:3). Compound **16a** was obtained as a white solid (23 mg, 37%), with a 5% contamination of compound **15a.** ¹H-NMR (300 MHz, CDCl₃) δ: 7.60 (d, *J* = 8.2 Hz, 1H), 7.48-7.35 (m, 3H), 7.34-7.27 (m, 2H), 7.21 (d, *J* = 2.2 Hz, 1H), 5.11 (*d*, *J* = 13.8 Hz. 1H), 3.59 (*d*, *J* = 13.9 Hz, 1H), 3.55 (s, 3 H), 2.43-2.21 (m, 1H), 2.20-1.98 (m, 2H), 1.98-1.82 (m, 1H), 1.82-1.64 (m, 2H). ¹³C-NMR (75 MHz, CDCl₃) δ: 173.4, 172.5, 151.7, 146.2, 133.5, 133.3, 129.0, 128.7, 128.2, 128.1, 127.2, 125.4, 124.6, 122.0, 113.3, 109.2, 54.7, 51.4, 33.6, 32.8, 20.5. HRMS (ESI+) *m*/*z* calcd for C₂₁H₁₉ClNO₃ [M+H]⁺ 368.1054, found 368.1043.

### Example 39: Methyl 5-(8-bromodidehydrodibenzo[b,f]azocin-5(6H)-yl)-5-oxopentanoate (16c)

Compound **15cX** (75 mg, 0.13 mmol) was dissolved in dry THF in a flame-dried flask under Ar-atmosphere, and the solution was cooled to -40 °C. Next, a solution of KO*^{t}*Bu in THF (1M, 260 µL, 0.26 mmol) was added dropwise. After 2 hours, only one bromide was eliminated, whereupon additional KO*^{t}*Bu (130 µL, 0.13 mmol) was added. After each subsequent hour an additional amount of KO*^{t}*Bu (30 µL, 0.03 mmol) was added, while maintaining the reaction at -40 °C. After 5.5 hours the reaction was completed and quenched by the addition of H₂O (5 mL). The H₂O-layer was extracted with EtOAc (3 × 10 mL). The combined organic layers were washed with H₂O (20 mL), and brine (20 mL) and subsequently dried over MgSO₄. The solvents were removed under reduced pressure and the crude product was purified by gradient column chromatography (EtOAc/*n*-heptane, 1:4 to 1:2) to obtain compound **16c** as a brown oil (20 mg, 37%). ¹H-NMR (400 MHz, CDCl₃) δ: 7.84 (d, *J* = 2.0 Hz, 1H), 7.44 (ddd, *J* = 8.1, 2.0, 0.4 Hz, 1H), 7.42-7.36 (m, 3H), 7.35-7.30 (m, 1H), 7.10 (d, *J* = 7.6 Hz, 1H), 5.08 (d, *J* = 13.9 Hz, 1H), 3.61 (d, *J* = 13.9 Hz, 1H), 3.56 (s, 3H), 2.36-2.24 (m, 1H), 2.21-2.05 (m, 2H), 1.97-1.85 (m, 1H), 1.83-1.69 (m, 2H). ¹³C-NMR (75 MHz, CDCl₃) δ: 173.4, 172.5, 151.5, 149.6, 135.3, 131.0, 129.0, 128.6, 128.2, 127.1, 126.5, 122.5, 122.3, 122.0, 113.9, 108.8, 54.8, 51.4, 33.6, 32.8, 20.5. HRMS (ESI+) *m*/*z* calcd for C₂₁H₁₈BrNNaO₃ [M+Na]⁺ 434.0368, found 434.0366.

### Example 40: Kinetic experiments

Kinetic experiments for **3**, **16a** and 16c were performed as follows. First, 2.25 µmol alkyne was mixed with 2.25 µmol benzylazide in 0.5 mL CD₃OD. The exact ratio between benzyl azide and alkyne was determined by comparison of the integrals of the aromatic signals and the benzylic protons of the alkyne. For **3**, **16a** and **16c,** the rate constant was determined by comparing the signal from the methyl ester of the starting material (δ: 3.52 ppm), to the signal from the methyl-ester of the product (δ: 3.60 ppm). Product formation was confirmed by mass spectrometry. For **16a:** HRMS (ESI+) *m*/*z* calcd for C₂₈H₂₆ClN₄O₃ [M+H]⁺ 501.1693, found 501.1693. For **16c**: HRMS (ESI+) *m*/*z* calcd for C₂₈H₂₆BrN₄O₃ [M+H]⁺ 545.1188, found 545.1189.

**Table 1. Rate constants of DIBAC and analogues in CD₃OD.**

| **Entry** | **Rate constant (M⁻¹s⁻¹)** |
|---|---|
| DIBAC (3) | 0.4 ± 0.1 |
| Cl-DIBAC (16a) | 0.9 ± 0.1 |
| Br-DIBAC (16c) | 0.80 ± 0.05 |

## Claims

1. Compound of the Formula (5): 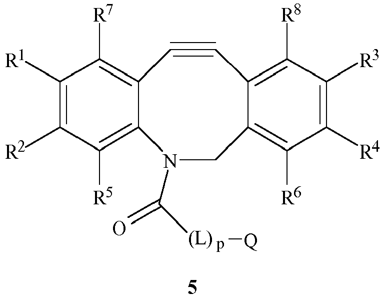 wherein:
R¹, R², R³, R⁴, R⁵, R⁶, R⁷ and R⁸ are independently selected from the group consisting of hydrogen, halogen, C₁ - C₁₂ haloalkyl, -CN, -N₃, -NO₂, -NCX, -XCN, -N(R⁹)₂, -N⁺(R⁹)₃, -C(X)N(R⁹)₂, -C(X)R⁹, -C(X)XR⁹, -S(O)R⁹, -S(O)₂R⁹, -S(O)OR⁹, -S(O)₂OR⁹, -S(O)N(R⁹)₂, -S(O)₂N(R⁹)₂, -OS(O)R⁹, -OS(O)₂R⁹, -OS(O)OR⁹, -OS(O)₂OR⁹, -P(O)(R⁹)(OR⁹), -P(O)(OR⁹)₂, -OP(O)(OR⁹)₂, -XC(X)R⁹, -XC(X)XR⁹, -XC(X)N(R⁹)₂, -N(R⁹)C(X)R⁹, -N(R⁹)C(X)XR⁹ and -N(R⁹)C(X)N(R⁹)₂, wherein X is oxygen or sulfur and wherein R⁹ is independently selected from the group consisting of hydrogen, halogen, C₁ - C₂₄ alkyl groups, C₂ - C₂₄ (hetero)aryl groups, C₃ - C₂₄ alkyl(hetero)aryl groups and C₃ - C₂₄ (hetero)arylalkyl groups;
with the proviso that at least one of R¹, R², R³, R⁴, R⁵, R⁶, R⁷ and R⁸ is selected from the group consisting of halogen, C₁ - C₁₂ haloalkyl-CN, -N₃, -NO₂, -NCX, -XCN, -N(R⁹)₂, -N⁺(R⁹)₃, -C(X)N(R⁹)₂, -C(X)R⁹, -C(X)XR⁹, -S(O)R⁹, -S(O)₂R⁹, -S(O)OR⁹, -S(O)₂OR⁹, -S(O)N(R⁹)₂, -S(O)₂N(R⁹)₂, -OS(O)R⁹, -OS(O)₂R⁹, -OS(O)OR⁹, -OS(O)₂OR⁹, -P(O)(R⁹)(OR⁹), -P(O)(OR⁹)₂, -OP(O)(OR⁹)₂, -XC(X)R⁹, -XC(X)XR⁹, -XC(X)N(R⁹)₂, -N(R⁹)C(X)R⁹, -N(R⁹)C(X)XR⁹ and -N(R⁹)C(X)N(R⁹)₂, wherein X and R⁹ are as defined above;
p is 0 or 1;
L is a linking group selected from the group consisting of linear or branched C₁-C₂₀₀ alkylene groups, C₂-C₂₀₀ alkenylene groups, C₂-C₂₀₀ alkynylene groups, C₃-C₂₀₀ cycloalkylene groups, C₅-C₂₀₀ cycloalkenylene groups, C₈-C₂₀₀ cycloalkynylene groups, C₂-C₂₀₀ (hetero)arylene groups, C₃-C₂₀₀ alkyl(hetero)arylene groups, C₃-C₂₀₀ (hetero)arylalkylene groups, C₄-C₂₀₀ (hetero)arylalkenylene groups, C₅-C₂₀₀ (hetero)arylalkynylene groups, the alkylene groups, alkenylene groups, alkynylene groups, cycloalkylene groups, cycloalkenylene groups, cycloalkynylene groups, (hetero)arylene groups, alkyl(hetero)arylene groups, (hetero)arylalkylene groups, (hetero)arylalkenylene groups, (hetero)arylalkynylene groups optionally being substituted and/or optionally interrupted by one or more heteroatoms, preferably 1 to 100 heteroatoms, said heteroatoms preferably being selected from the group consisting of O, S, N and NR¹², wherein R¹² is independently selected from the group consisting of hydrogen, halogen, C₁ - C₂₄ alkyl groups, C₂ - C₂₄ (hetero)aryl groups, C₃ - C₂₄ alkyl(hetero)aryl groups and C₃ - C₂₄ (hetero)arylalkyl groups; and
Q is a functional group selected from the group consisting of hydrogen, halogen, R¹¹, -CH=C(R¹¹)₂, -C≡CR¹¹, -[C(R¹¹)₂C(R¹¹)₂O]_{q}-R¹¹ wherein q is in the range of 1 to 200, -CN, -N₃, -NCX, -XCN, -XR¹¹, -N(R¹¹)₂, -N⁺(R¹¹)₃, -C(X)N(R¹¹)₂, -C(R¹¹)₂XR¹¹, -C(X)R¹¹, -C(X)XR¹¹, -S(O)R¹¹, -S(O)₂R¹¹, -S(O)OR¹¹, -S(O)₂OR¹¹, -S(O)N(R¹¹)₂, -S(O)₂N(R¹¹)₂, -OS(O)R¹¹, -OS(O)₂R¹¹, -OS(O)OR¹¹, -OS(O)₂OR¹¹, -P(O)(R¹¹)(OR¹¹), -P(O)(OR¹¹)₂, -OP(O)(OR¹¹)₂, -Si(R¹¹)₃, -XC(X)R¹¹, -XC(X)XR¹¹, -XC(X)N(R¹¹)₂, -N(R¹¹)C(X)R¹¹, -N(R¹¹)C(X)XR¹¹ and -N(R¹¹)C(X)N(R¹¹)₂, wherein X is oxygen or sulfur and wherein R¹¹ is independently selected from the group consisting of hydrogen, halogen, C₁ - C₂₄ alkyl groups, C₂ - C₂₄ (hetero)aryl groups, C₃ - C₂₄ alkyl(hetero)aryl groups and C₃ - C₂₄ (hetero)arylalkyl groups.

2. Compound according to claim 1, wherein R⁷ and R⁸ are hydrogen.

3. Compound according to claim 1 or claim 2, wherein R⁵ and R⁶ are hydrogen.

4. Compound according to any one of the preceding claims, wherein R¹ is equal to R³, R² is equal to R⁴, R⁷ is equal to R⁸ and R⁵ is equal to R⁶.

5. Compound according to any one of the preceding claims, wherein R¹ and R³ are selected from the group consisting of F, Cl and Br.

6. Compound according to any one of the preceding claims, wherein R² and R⁴ are selected from the group consisting of F, Cl and Br.

7. Compound according to any one of claims 1 - 3, wherein R³ is selected from the group consisting of F, Cl and Br, and wherein R¹, R² and R⁴ are hydrogen.

8. Compound according to any one of claims 1 - 3, wherein R² and R⁴ are independently selected from the group consisting of F, Cl and Br, and wherein R¹ and R³ are hydrogen.

9. Compound according to any one of the preceding claims, wherein p is 1 and L is a linear or branched C₁ - C₂₄ alkylene group.

10. Compound according to any one of the preceding claims, wherein Q is selected from the group consisting of hydrogen, halogen, -OR¹¹, -SR¹¹, -N(R¹¹)₂, -N⁺(R¹¹)₃, -C(O)N(R¹¹)₂, -C(O)OR¹¹, -OC(O)R¹¹, -OC(O)OR¹¹, -OC(O)N(R¹¹)₂, -N(R¹¹)C(O)R¹¹, -N(R¹¹)C(O)OR¹¹ and -N(R¹¹)C(O)N(R¹¹)₂, wherein R¹¹ is as defined in claim 1.

11. Conjugate wherein a compound according to any one of claims 1 - 10 is conjugated to a label via a functional group Q, wherein the label is selected from the group consisting of fluorophores, biotin, polyethylene glycol chains, polypropylene glycol chains, mixed polyethylene/polypropylene glycol chains, metal chelator complexes, radioactive isotopes, steroids, pharmaceutical compounds, lipids, amino acids, peptides, polypeptides, glycans, nucleotides, peptide tags and solid phases.

12. Method for the modification of a target molecule, wherein a conjugate according to claim 11 is reacted with a compound comprising a 1,3-dipole or a 1,3-(hetero)diene.

13. Method according to claim 12, wherein the compound comprising a 1,3-dipole is an azide-comprising compound, a nitrone-comprising compound or a nitrile oxide-comprising compound.

14. Use of a conjugate according to claim 11 for bioorthogonal labeling, imaging or modification of a target molecule.

15. Composition comprising a conjugate according to claim 11, further comprising a pharmaceutically acceptable carrier.

## Patentansprüche

1. Verbindung der Formel (5): 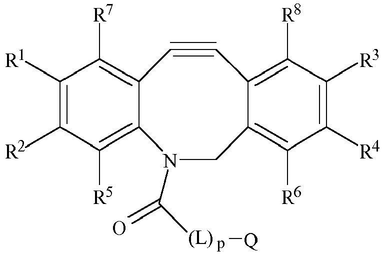
wobei R¹, R², R³, R⁴, R⁵, R⁶, R⁷, und R⁸ unabhängig voneinander ausgewählt sind aus der Gruppe bestehend aus Wasserstoff, Halogen, C₁-C₁₂ Haloalkyl, -CN, -N₃, -NO₂, -NCX, -XCN, -N(R⁹)₂, -N⁺(R⁹)₃, -C(X)N(R⁹)₂, -C(X)R⁹, -C(X)XR⁹, -S(O)R⁹, -S(O)₂R⁹, -S(O)OR⁹, -S(O)₂OR⁹, -S(O)N(R⁹)₂, -S(O)₂N(R⁹)₂, -OS(O)R⁹, -OS(O)₂R⁹, -OS(O)OR⁹, -OS(O)₂OR⁹, -P(O)(R⁹)(OR⁹), -P(O)(OR⁹)₂, -OP(O)(OR⁹)₂, -XC(X)R⁹, -XC(X)XR⁹, -XC(X)N(R⁹)₂, -N(R⁹)C(X)R⁹, -N(R⁹)C(X)XR⁹ und -N(R⁹)C(X)N(R⁹)₂, wobei X Sauerstoff oder Schwefel ist und R⁹ unabhängig ausgewählt ist aus der Gruppe bestehend aus Wasserstoff, Halogen, C₁ - C₂₄ Alkylgruppen, C₂ - C₂₄ (Hetero)Arylgruppen, C₃ - C₂₄ Alkyl(hetero)arylgruppen und C₃ - C₂₄ (Hetero)Arylalkylgruppen;
unter der Maßgabe, dass mindestens einer der Reste R¹, R², R³, R⁴, R⁵, R⁶, R⁷, und R⁸ ausgewählt ist aus der Gruppe bestehend aus Halogen, C₁-C₁₂ Haloalkyl, -CN, -N₃, -NO₂, -NCX, -XCN, -N(R⁹)₂, -N⁺(R⁹)₃, -C(X)N(R⁹)₂, -C(X)R⁹, -C(X)XR⁹, -S(O)R⁹, -S(O)₂R⁹, -S(O)OR⁹, -S(O)₂OR⁹, -S(O)N(R⁹)₂, -S(O)₂N(R⁹)₂, -OS(O)R⁹, -OS(O)₂R⁹, -OS(O)OR⁹, -OS(O)₂OR⁹, -P(O)(R⁹)(OR⁹), -P(O)(OR⁹)₂, -OP(O)(OR⁹)₂, -XC(X)R⁹, -XC(X)XR⁹, -XC(X)N(R⁹)₂, -N(R⁹)C(X)R⁹, -N(R⁹)C(X)XR⁹ und -N(R⁹)C(X)N(R⁹)₂, wobei X und R⁹ wie oben definiert sind;
p 0 oder 1 ist;
L eine Verbindungsgruppe ist, ausgewählt aus der Gruppe bestehend aus linearen oder verzweigten C₁ - C₂₀₀ Alkylengruppen, C₂ - C₂₀₀ Alkenylengruppen, C₂ - C₂₀₀ Alkynylengruppen, C₃ - C₂₀₀ Cycloalkylengruppen, C₅ - C₂₀₀ Cycloalkenylengruppen, C₈ - C₂₀₀ Cycloalkynylengruppen, C₂ - C₂₀₀ (Hetero)Arylengruppen, C₃ - C₂₀₀ Alkyl(hetero)arylengruppen, C₃ - C₂₀₀ (Hetero)Arylalkylengruppen, C₄ - C₂₀₀ (Hetero)Arylalkenylengruppen, C₅ - C₂₀₀ (Hetero)Arylalkynylengruppen, den Alkylengruppen, Alkenylengruppen, Alkynylengruppen, Cycloalkylengruppen, Cycloalkenylengruppen, Cycloalkynylengruppen, (Hetero)Arylengruppen, Alkyl(hetero)arylengruppen, (Hetero)Arylalkylengruppen, (Hetero)Arylalkenylengruppen, (Hetero)Arylalkynylengruppen, optional substituiert und / oder optional unterbrochen durch ein oder mehrere Heteroatome, bevorzugt 1 bis 100 Heteroatome, besagte Heteroatome sind bevorzugt ausgewählt aus der Gruppe bestehend aus O, S, N und NR¹², wobei R¹² unabhängig ausgewählt ist aus der Gruppen bestehend aus Wasserstoff, Halogen, C₁ - C₂₄ Alkylgruppen, C₂ - C₂₄ (Hetero)Arylgruppen, C₃ - C₂₄ Alkyl(hetero)arylgruppen und C₃ - C₂₄ (Hetero)Arylalkylgruppen; und
Q eine funktionelle Gruppe ist, ausgewählt aus der Gruppe bestehend aus Wasserstoff, Halogen, R¹¹, -CH=C(R¹¹)₂, -C=CR¹¹, -[C(R¹¹)₂C(R¹¹)₂O]_{q}-R¹¹ wobei q in einem Bereich liegt zwischen 1 und 200, -CN, -N₃, -NCX, -XCN, -XR¹¹, -N(R¹¹)₂, -N⁺(R¹¹)₃, -C(X)N(R¹¹)₂, -C(R¹¹)₂XR¹¹, -C(X)R¹¹, -C(X)XR¹¹, -S(O)R¹¹, -S(O)₂R¹¹, -S(O)OR¹¹, -S(O)₂OR¹¹, -S(O)N(R¹¹)₂, -S(O)₂N(R¹¹)₂, -OS(O)R¹¹, -OS(O)₂R¹¹, -OS(O)OR¹¹, -OS(O)₂OR¹¹, -P(O)(R¹¹)(OR¹¹), -P(O)(OR¹¹)₂, -OP(O)(OR¹¹)₂, -Si(R¹¹)₃, -XC(X)R¹¹, -XC(X)XR¹¹, -XC(X)N(R¹¹)₂, -N(R¹¹)C(X)R¹¹, -N(R¹¹)C(X)XR¹¹ und -N(R¹¹)C(X)N(R¹¹)₂, wobei X Sauerstoff oder Schwefel ist und wobei R¹¹ unabhängig ausgewählt ist aus der Gruppe bestehend aus Wasserstoff, Halogen, C₁ - C₂₄ Alkylgruppen, C₂ - C₂₄ (Hetero)Arylgruppen, C₃ - C₂₄ Alkyl(hetero)arylgruppen und C₃ - C₂₄ (Hetero)Arylalkylgruppen.

2. Verbindung nach Anspruch 1, wobei R⁷ und R⁸ Wasserstoff sind.

3. Verbindung nach Anspruch 1 oder Anspruch 2, wobei R⁵ und R⁶ Wasserstoff sind

4. Verbindung nach einem der vorangehenden Ansprüche, wobei R¹ und R³ gleich sind, R² und R⁴ gleich sind, R⁷ und R⁸ gleich sind und R⁵ und R⁶ gleich sind.

5. Verbindung nach einem der vorangehenden Ansprüche, wobei R¹ und R³ ausgewählt sind aus der Gruppe bestehend aus F, Cl und Br.

6. Verbindung nach einem der vorangehenden Ansprüche, wobei R² und R⁴ ausgewählt sind aus der Gruppe bestehend aus F, Cl und Br.

7. Verbindung nach einem der Ansprüche 1 bis 3, wobei R³ ausgewählt ist aus der Gruppe bestehend aus F, Cl und Br, und wobei R¹, R² und R⁴ Wasserstoff sind.

8. Verbindung nach einem der Ansprüche 1 bis 3, wobei R² und R⁴ unabhängig ausgewählt sind aus der Gruppe bestehend aus F, Cl und Br, und wobei R¹ und R³ Wasserstoff sind.

9. Verbindung nach einem der vorangehenden Ansprüche, wobei p 1 ist und L eine lineare oder verzweigte C₁ - C₂₄ Alkylengruppe ist.

10. Verbindung nach einem der vorangehenden Ansprüche, wobei Q ausgewählt ist aus der Gruppe bestehend aus Wasserstoff, Halogen, -OR¹¹, -SR¹¹, -N(R¹¹)₂, -N⁺(R¹¹)₃ -C(O)N(R¹¹)₂, -C(O)OR¹¹, -OC(O)R¹¹, -OC(O)OR¹¹, -OC(O)N(R¹¹)₂, -N(R¹¹)C(O)R¹¹, -N(R¹¹)C(O)OR¹¹ und -N(R¹¹)C(O)N(R¹¹)₂, wobei R¹¹ wie in Anspruch 1 definiert ist.

11. Konjugat, worin eine Verbindung nach einem der Ansprüche 1 bis 10 über eine funktionelle Gruppe Q konjugiert ist mit einem Marker, wobei der Marker ausgewählt ist aus der Gruppe bestehend aus Fluorophoren, Biotin, Polyethylenglycolketten, Polypropylenglycolketten, gemischte Polyethylen/Polypropylenglycolketten, Metall-Chelator-Komplexen, radioaktiven Isotopen, Steroiden, pharmazeutischen Verbindungen, Lipiden, Aminosäuren, Peptiden, Polypeptiden, Glykane, Nukleotide, Peptid-Tags und festen Phasen.

12. Verfahren zur Modifizierung eines Zielmoleküls, wobei ein Konjugat nach Anspruch 11 mit einer Verbindung umfassend einen 1,3-Dipol oder ein 1,3-(Hetero)Dien reagiert wird.

13. Verfahren nach Anspruch 12, wobei die Verbindung umfassend einen 1,3-Dipol eine Azid umfassende Verbindung, eine Nitron umfassende Verbindung oder eine Nitriloxid umfassende Verbindung ist.

14. Verwendung eines Konjugats nach Anspruch 11 zur bioorthogonalen Markierung, Bildgebung oder Modifizierung eines Zielmoleküls.

15. Mischung umfassend ein Konjugat nach Anspruch 11, sowie umfassend einen pharmazeutisch annehmbaren Träger.

## Revendications

1. Composé de formule (5) : 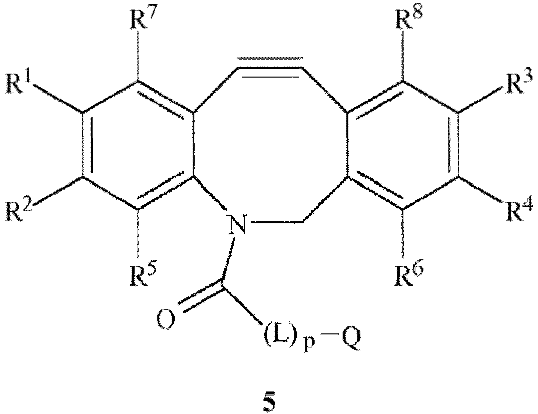 dans lequel :
R¹, R², R³, R⁴, R⁵, R⁶, R⁷ et R⁸ sont choisis indépendamment dans le groupe comprenant hydrogène, halogène, haloalkyle en C₁-C₁₂, -CN, -N₃, -NO₂, -NCX, -XCN, -N(R⁹)₂, -N⁺(R⁹)₃, -C(X)N(R⁹)₂, -C(X)R⁹, -C(X)XR⁹, -S(O)R⁹, -S(O)₂R⁹, -S(O)OR⁹, -S(O)₂OR⁹, -S(O)N(R⁹)₂, -S(O)₂N(R⁹)₂, -OS(O)R⁹, -OS(O)₂R⁹, -OS(O)OR⁹, -OS(O)₂OR⁹, -P(O)(R)⁹(OR⁹), -P(O)(OR⁹)₂, -OP(O)(OR⁹)₂, -XC(X)R⁹, -XC(X)XR⁹, -XC(X)N(R⁹)₂, -N(R⁹)C(X)R⁹, -N(R⁹)C(X)XR⁹ et -N(R⁹)C(X)N(R⁹)₂, dans lequel X représente oxygène ou soufre et dans lequel R⁹ est choisi indépendamment dans le groupe comprenant hydrogène, halogène, des groupes alkyle en C₁-C₂₄, des groupes (hétéro)aryle en C₂-C₂₄, des groupes alkyl(hétéro)aryle en C₃-C₂₄ et des groupes (hétéro)arylalkyle en C₃-C₂₄ ;
à condition qu'au moins l'un de R¹, R², R³, R⁴, R⁵, R⁶, R⁷ et R⁸ soit choisis dans le groupe comprenant halogène, haloalkyle en C₁-C₁₂, -CN, -N₃, -NO₂, - NCX, -XCN, -N(R⁹)₂, -N⁺(R⁹)₃, -C(X)N(R⁹)₂, -C(X)R⁹, -C(X)XR⁹, -S(O)R⁹, -S(O)₂R⁹, -S(O)OR⁹, -S(O)₂OR⁹, -S(O)N(R⁹)₂, -S(O)₂N(R⁹)₂, -OS(O)R⁹, -OS(O)₂R⁹, -OS(O)OR⁹, -OS(O)₂OR⁹, -P(O)(R)⁹(OR⁹), -P(O)(OR⁹)₂, -OP(O)(OR⁹)₂, -XC(X)R⁹, -XC(X)XR⁹, -XC(X)N(R⁹)₂, -N(R⁹)C(X)R⁹, -N(R⁹)C(X)XR⁹ et -N(R⁹)C(X)N(R⁹)₂, dans lequel X et R⁹ sont tels que définis ci-dessus ;
p est 0 ou 1 ;
L est un groupe de liaison choisi dans le groupe constitué par des groupes alkylène en C₁-C₂₀₀ linéaires ou ramifiés, des groupes alcénylène en C₂-C₂₀₀, des groupes alcynylène en C₂-C₂₀₀, des groupes cycloalkylène en C₃-C₂₀₀, des groupes cycloalcénylène en C₅-C₂₀₀, des groupe cycloalcynylène en C₈-C₂₀₀, des groupes (hétéro)arylène en C₂-C₂₀₀, des groupes alkyl(hétéro)arylène en C₃-C₂₀₀, des groupes (hétéro)arylalkylène en C₃-C₂₀₀, des groupes (hétéro)arylalcénylène en C₄-C₂₀₀, des groupes (hétéro)arylalcynylène en C₅-C₂₀₀, des groupes alkylènes, des groupes alcénylène, des groupes alcynylène, des groupes cycloalkylène, des groupes cycloalcénylène, des groupes cyclloalcynylène, des groupes (hétéro)arylène, des groupes alkyl(hétéro)arylène, des groupes (hétéro)arylakylène, des groupes (hétéro)arylacénylène, des groupes (hétéro)arylalcynylène étant facultativement substitués et/ou éventuellement interrompus par un ou plusieurs hétéroatomes, de préférence 1 à 100 hétéroatomes, lesdits hétéroatomes étant de préférence choisis dans le groupe comprenant O, S, N et NR¹², dans lequel R¹² est sélectionné indépendamment dans le groupe comprenant hydrogène, halogène, des groupes alkyle en C₁-C₂₄, des groupes (hétéro)aryle en C₂-C₂₄, des groupes alkyl(hétéro)aryle en C₃-C₂₄ et des groupes (hétéro)arylalkyle en C₃-C₂₄ ;
et
Q est un groupe fonctionnel choisi dans le groupe comprenant hydrogène, halogène, R¹¹, -CH=C(R¹¹)₂, -C=CR¹¹, -[C(R¹¹)₂C(R¹¹)₂O]_{q}R¹¹, dans lequel q est dans la plage de 1 à 200, -CN, -N₃, -NCX, -XCN, -XR¹¹, -N(R¹¹)₂, -N⁺(R¹¹)₃, -C(X)N(R¹¹)₂, -C(R¹¹)₂XR¹¹, -C(X)R¹¹, -C(X)XR¹¹, -S(O)R¹¹, -S(O)₂R¹¹, -S(O)OR¹¹, -S(O)₂OR¹¹, -S(O)N(R¹¹)₂, -S(O)₂N(R¹¹)₂, -OS(O)R¹¹, -OS(O)₂R¹¹, -OS(O)OR¹¹, -OS(O)₂OR¹¹, -P(O)(R¹¹)(OR¹¹), -P(O)(OR¹¹)₂, -OP(O)(OR¹¹)₂, -Si(R¹¹)₃, -XC(X)R¹¹, -XC(X)XR¹¹, -XC(X)N(R¹¹)₂, -N(R¹¹)C(X)R¹¹, -N(R¹¹)C(X)XR¹¹ et -N(R¹¹)C(X)N(R¹¹)₂, dans lequel X est oxygène ou soufre et dans lequel R¹¹ est choisi indépendamment dans le groupe comprenant hydrogène, halogène, des groupes alkyle en C₁-C₂₄, des groupes hétéroaryle en C₂-C₂₄, des groupes alkyl(hétéro)aryl en C₃-C₂₄ et des (hétéro)arylalkyle en C₃-C₂₄.

2. Composé selon la revendication 1, dans lequel R⁷ et R⁸ sont de l'hydrogène.

3. Composé selon la revendication 1 ou la revendication 2, dans lequel R⁵ et R⁶ sont de l'hydrogène.

4. Composé selon l'une quelconque des revendications précédentes, dans lequel R¹ est égal à R³, R² est égal à R⁴, R⁷ est égal à R⁸ et R⁵ est égal à R⁶.

5. Composé selon l'une quelconque des revendications précédentes, dans lequel R¹ et R³ sont choisis dans le groupe comprenant F, Cl et Br.

6. Composé selon l'une quelconque des revendications précédentes, dans lequel R² et R⁴ sont choisis dans le groupe comprenant F, Cl et Br.

7. Composé selon l'une quelconque des revendications 1 à 3, dans lequel R³ est choisi dans le groupe comprenant F, Cl et Br, et dans lequel R¹, R² et R⁴ sont de l'hydrogène.

8. Composé selon l'une quelconque des revendications 1 à 3, dans lequel R² et R⁴ sont choisis indépendamment dans le groupe comprenant F, Cl et Br, et dans lequel R¹ et R³ sont de l'hydrogène.

9. Composé selon l'une quelconque des revendications précédentes, dans lequel p est 1 et L est un groupe alkylène en C₁-C₂₄ linéaire ou ramifié.

10. Composé selon l'une quelconque des revendications précédentes, dans lequel Q est choisi dans le groupe comprenant hydrogène, halogène, -OR¹¹, -SR¹¹, -N(R¹¹)₂, -N⁺(R¹¹)₃, -C(O)N(R¹¹)₂, -C(O)OR¹¹, -OC(O)R¹¹, -OC(O)OR¹¹, -OC(O)N(R¹¹)₂, -N(R¹¹)C(O)R¹¹. -N(R¹¹)C(O)OR¹¹ et -N(R¹¹)C(O)N(R¹¹)₂, dans lequel R¹¹ est tel que défini dans la revendication 1.

11. Conjugué dans lequel un composé selon l'une quelconque des revendications 1 à 10 est conjugué à une label via un groupe fonctionnel Q, dans lequel le label est choisie dans le groupe comprenant des fluorophores, de la biotine, des chaînes de polyéthylène glycol, des chaînes de polypropylène glycol, des chaînes de polyéthylène/polypropylène glycol mélangées, des complexes chélateurs métalliques, des isotopes radioactifs, des stéroïdes, des composés pharmaceutiques, des lipides, des acides aminés, des peptides, des polypeptides, des glycanes, des nucléotides, des étiquettes peptidiques et des phases solides.

12. Procédé pour la modification d'une molécule cible, dans lequel un conjugué selon la revendication 11 est mis à réagir avec un composé comprenant un 1,3-dipôle ou un 1,3-(hétéro)diène.

13. Procédé selon la revendication 12, dans lequel le composé comprenant un 1,3-dipôle est un composé comprenant un azide, un composé comprenant une nitrone, ou un composé comprenant de l'oxyde de nitrile.

14. Utilisation d'un conjugué selon la revendication 11 pour un étiquetage bioorthogonal, une imagerie ou une modification d'une molécule cible.

15. Composition comprenant un conjugué selon la revendication 11, comprenant en outre un support pharmaceutiquement acceptable.
